# EUROPEAN PATENT SPECIFICATION

(11) **EP 2 971 075 B1**
(45) Date of publication and mention of the grant of the patent: **15.08.2018**
(21) Application number: 14709926.1
(22) Date of filing: 12.03.2014
(51) Int. Cl.: C12Q 1/68

(54) **METHODS AND COMPOSITIONS FOR DETECTING MUTATIONS IN THE HUMAN PI3KCA (PIK3CA) GENE**
VERFAHREN UND ZUSAMMENSETZUNGEN ZUM NACHWEIS VON MUTATIONEN IM MENSCHLICHEN PI3KCA (PIK3CA)-GEN
PROCÉDÉS ET COMPOSITIONS POUR LA DÉTECTION DE MUTATIONS DANS LE GÈNE PI3KCA HUMAIN (PIK3CA)

(30) Priority: 13.03.2013 US 201361780017 P
(43) Date of publication of application: 20.01.2016
(73) Proprietor: Roche Diagnostics GmbH, 68305 Mannheim (DE); F. Hoffmann-La Roche AG, 4070 Basel (CH)
(72) Inventor: TSAN, Alison, Danville California 94526 (US)
(74) Representative: Schwarz, Ralf
(86) International application number: PCT/EP2014/054766
(87) International publication number: WO 2014/140061

(56) References cited:
- WO-A1-2011/087928
- WO-A1-2011/131151
- WO-A2-2009/040557
- WO-A2-2009/040557
- WO-A2-2009/040557
- US-A1- 2010 286 143
- US-A1- 2010 286 143
- L. H. SAAL: "PIK3CA Mutations Correlate with Hormone Receptors, Node Metastasis, and ERBB2, and Are Mutually Exclusive with PTEN Loss in Human Breast Carcinoma", CANCER RESEARCH, vol. 65, no. 7, 1 April 2005 (2005-04-01), pages 2554-2559, XP055070087, ISSN: 0008-5472, DOI: 10.1158/0008-5472-CAN-04-3913
- KATHERINE STEMKE-HALE ET AL: "Frequency of mutations and polymorphisms in borderline ovarian tumors of known cancer genes", MODERN PATHOLOGY, vol. 26, no. 4, 23 November 2012 (2012-11-23), pages 544-552, XP055332175, GB ISSN: 0893-3952, DOI: 10.1038/modpathol.2012.194
- MURRAY S ET AL: "Low frequency of somatic mutations in uterine sarcomas: A molecular analysis and review of the literature", MUTATION RESEARCH, ELSEVIER, AMSTERDAM, NL, vol. 686, no. 1-2, 1 April 2010 (2010-04-01), pages 68-73, XP026944638, ISSN: 0027-5107, DOI: 10.1016/J.MRFMMM.2010.01.019 [retrieved on 2010-02-01]
- M. GYMNOPOULOS ET AL: "Rare cancer-specific mutations in PIK3CA show gain of function", PROCEEDINGS OF THE NATIONAL ACADEMY OF SCIENCES, vol. 104, no. 13, 27 March 2007 (2007-03-27), pages 5569-5574, XP055119028, ISSN: 0027-8424, DOI: 10.1073/pnas.0701005104
- L. H. SAAL: "PIK3CA Mutations Correlate with Hormone Receptors, Node Metastasis, and ERBB2, and Are Mutually Exclusive with PTEN Loss in Human Breast Carcinoma", CANCER RESEARCH, vol. 65, no. 7, 1 April 2005 (2005-04-01), pages 2554-2559, XP055070087, ISSN: 0008-5472, DOI: 10.1158/0008-5472-CAN-04-3913
- KATHERINE STEMKE-HALE ET AL: "Frequency of mutations and polymorphisms in borderline ovarian tumors of known cancer genes", MODERN PATHOLOGY, vol. 26, no. 4, 23 November 2012 (2012-11-23), pages 544-552, XP055332175, GB ISSN: 0893-3952, DOI: 10.1038/modpathol.2012.194
- MURRAY S ET AL: "Low frequency of somatic mutations in uterine sarcomas: A molecular analysis and review of the literature", MUTATION RESEARCH, ELSEVIER, AMSTERDAM, NL, vol. 686, no. 1-2, 1 April 2010 (2010-04-01), pages 68-73, XP026944638, ISSN: 0027-5107, DOI: 10.1016/J.MRFMMM.2010.01.019 [retrieved on 2010-02-01]

### SEQUENCE LISTING

<110> ROCHE DIAGNOSTICS GMBH
   F. HOFFMANN-LA ROCHE AG
<120> METHODS AND COMPOSITIONS FOR DETECTING MUTATIONS IN THE HUMAN PI3KCA (PIK3CA) GENE
<130> 31384 WO-KOE
<150> 61/780,017
   <151> 2013-03-13
<160> 230
<170> PatentIn version 3.5
<210> 1
   <211> 23
   <212> DNA
   <213> Artificial Sequence
<220>
   <221> source
   <223> /note="Description of Artificial Sequence: Synthetic primer"
<400> 1
   ttttgttgtc cagccaccat gat 23
<210> 2
   <211> 23
   <212> DNA
   <213> Artificial Sequence
<220>
   <221> source
   <223> /note="Description of Artificial Sequence: Synthetic primer"
<400> 2
   ttttgttgtc cagccaccat gaa 23
<210> 3
   <211> 23
   <212> DNA
   <213> Artificial Sequence
<220>
   <221> source
   <223> /note="Description of Artificial Sequence: Synthetic primer"
<400> 3
   ttttgttgtc cagccaccat gca 23
<210> 4
   <211> 23
   <212> DNA
   <213> Artificial Sequence
<220>
   <221> source
   <223> /note="Description of Artificial Sequence: Synthetic primer"
<400> 4
   ttttgttgtc cagccaccat gga 23
<210> 5
   <211> 23
   <212> DNA
   <213> Artificial Sequence
<220>
   <221> source
   <223> /note="Description of Artificial Sequence: Synthetic primer"
<400> 5
   ttttgttgtc cagccaccat gta 23
<210> 6
   <211> 23
   <212> DNA
   <213> Artificial Sequence
<220>
   <221> source
   <223> /note="Description of Artificial Sequence: Synthetic primer"
<400> 6
   ttttgttgtc cagccaccat caa 23
<210> 7
   <211> 23
   <212> DNA
   <213> Artificial Sequence
<220>
   <221> source
   <223> /note="Description of Artificial Sequence: Synthetic primer"
<400> 7
   ttttgttgtc cagccaccat taa 23
<210> 8
   <211> 23
   <212> DNA
   <213> Artificial Sequence
<220>
   <221> source
   <223> /note="Description of Artificial Sequence: Synthetic primer"
<400> 8
   ttttgttgtc cagccaccat aaa 23
<210> 9
   <211> 23
   <212> DNA
   <213> Artificial Sequence
<220>
   <221> source
   <223> /note="Description of Artificial Sequence: Synthetic primer"
<400> 9
   ttttgttgtc cagccaccaa gaa 23
<210> 10
   <211> 23
   <212> DNA
   <213> Artificial Sequence
<220>
   <221> source
   <223> /note="Description of Artificial Sequence: Synthetic primer"
<400> 10
   ttttgttgtc cagccaccac gaa 23
<210> 11
   <211> 23
   <212> DNA
   <213> Artificial Sequence
<220>
   <221> source
   <223> /note="Description of Artificial Sequence: Synthetic primer"
<400> 11
   ttttgttgtc cagccaccag gaa 23
<210> 12
   <211> 25
   <212> DNA
   <213> Artificial Sequence
<220>
   <221> source
   <223> /note="Description of Artificial Sequence: Synthetic primer"
<400> 12
   gtttttgttg tccagccacc atgaa 25
<210> 13
   <211> 25
   <212> DNA
   <213> Artificial Sequence
<220>
   <221> source
   <223> /note="Description of Artificial Sequence: Synthetic primer"
<220>
   <221> modified_base
   <222> (25)..(25)
   <223> N6-tert-butyl-benzyl-deoxyadenine
<400> 13
   gtttttgttg tccagccacc atgaa 25
<210> 14
   <211> 25
   <212> DNA
   <213> Artificial Sequence
<220>
   <221> source
   <223> /note="Description of Artificial Sequence: Synthetic primer"
<220>
   <221> modified_base
   <222> (24)..(24)
   <223> N6-tert-butyl-benzyl-deoxyadenine
<400> 14
   gtttttgttg tccagccacc atgaa 25
<210> 15
   <211> 27
   <212> DNA
   <213> Artificial Sequence
<220>
   <221> source
   <223> /note="Description of Artificial Sequence: Synthetic primer"
<220>
   <221> modified_base
   <222> (2)..(2)
   <223> N4-methyl-deoxycytosine
<220>
   <221> modified_base
   <222> (14)..(14)
   <223> N4-methyl-deoxycytosine
<220>
   <221> modified_base
   <222> (18)..(18)
   <223> N4-methyl-deoxycytosine
<220>
   <221> modified_base
   <222> (22)..(22)
   <223> N4-methyl-deoxycytosine
<220>
   <221> modified_base
   <222> (26)..(26)
   <223> N6-tert-butyl-benzyl-deoxyadenine
<400> 15
   ccgtttttgt tgtccagcca ccatgaa 27
<210> 16
   <211> 30
   <212> DNA
   <213> Artificial Sequence
<220>
   <221> source
   <223> /note="Description of Artificial Sequence: Synthetic primer"
<220>
   <221> modified_base
   <222> (5)..(5)
   <223> N4-methyl-deoxycytosine
<220>
   <221> modified_base
   <222> (17)..(17)
   <223> N4-methyl-deoxycytosine
<220>
   <221> modified_base
   <222> (21)..(21)
   <223> N4-methyl-deoxycytosine
<220>
   <221> modified_base
   <222> (25)..(25)
   <223> N4-methyl-deoxycytosine
<220>
   <221> modified_base
   <222> (30)..(30)
   <223> N6-tert-butyl-benzyl-deoxyadenine
<400> 16
   aatccattgt tgttgtccag ccaccatgaa 30
<210> 17
   <211> 22
   <212> DNA
   <213> Artificial Sequence
<220>
   <221> source
   <223> /note="Description of Artificial Sequence: Synthetic primer"
<400> 17
   tttgttgtcc agccaccatg at 22
<210> 18
   <211> 22
   <212> DNA
   <213> Artificial Sequence
<220>
   <221> source
   <223> /note="Description of Artificial Sequence: Synthetic primer"
<400> 18
   tttgttgtcc agccaccatg cc 22
<210> 19
   <211> 22
   <212> DNA
   <213> Artificial Sequence
<220>
   <221> source
   <223> /note="Description of Artificial Sequence: Synthetic primer"
<400> 19
   tttgttgtcc agccaccatg gc 22
<210> 20
   <211> 22
   <212> DNA
   <213> Artificial Sequence
<220>
   <221> source
   <223> /note="Description of Artificial Sequence: Synthetic primer"
<400> 20
   tttgttgtcc agccaccatg tc 22
<210> 21
   <211> 22
   <212> DNA
   <213> Artificial Sequence
<220>
   <221> source
   <223> /note="Description of Artificial Sequence: Synthetic primer"
<400> 21
   tttgttgtcc agccaccatc ac 22
<210> 22
   <211> 22
   <212> DNA
   <213> Artificial Sequence
<220>
   <221> source
   <223> /note="Description of Artificial Sequence: Synthetic primer"
<400> 22
   tttgttgtcc agccaccatt ac 22
<210> 23
   <211> 22
   <212> DNA
   <213> Artificial Sequence
<220>
   <221> source
   <223> /note="Description of Artificial Sequence: Synthetic primer"
<400> 23
   tttgttgtcc agccaccata ac 22
<210> 24
   <211> 22
   <212> DNA
   <213> Artificial Sequence
<220>
   <221> source
   <223> /note="Description of Artificial Sequence: Synthetic primer"
<400> 24
   tttgttgtcc agccaccaag ac 22
<210> 25
   <211> 22
   <212> DNA
   <213> Artificial Sequence
<220>
   <221> source
   <223> /note="Description of Artificial Sequence: Synthetic primer"
<400> 25
   tttgttgtcc agccaccacg ac 22
<210> 26
   <211> 22
   <212> DNA
   <213> Artificial Sequence
<220>
   <221> source
   <223> /note="Description of Artificial Sequence: Synthetic primer"
<400> 26
   tttgttgtcc agccaccagg ac 22
<210> 27
   <211> 22
   <212> DNA
   <213> Artificial Sequence
<220>
   <221> source
   <223> /note="Description of Artificial Sequence: Synthetic primer"
<400> 27
   tttgttgtcc agccaccatg at 22
<210> 28
   <211> 22
   <212> DNA
   <213> Artificial Sequence
<220>
   <221> source
   <223> /note="Description of Artificial Sequence: Synthetic primer"
<400> 28
   tttgttgtcc agccaccatg cc 22
<210> 29
   <211> 27
   <212> DNA
   <213> Artificial Sequence
<220>
   <221> source
   <223> /note="Description of Artificial Sequence: Synthetic primer"
<400> 29
   tttcatgaaa caaatgaatg atgcagg 27
<210> 30
   <211> 27
   <212> DNA
   <213> Artificial Sequence
<220>
   <221> source
   <223> /note="Description of Artificial Sequence: Synthetic primer"
<400> 30
   tttcatgaaa caaatgaatg atgcatg 27
<210> 31
   <211> 27
   <212> DNA
   <213> Artificial Sequence
<220>
   <221> source
   <223> /note="Description of Artificial Sequence: Synthetic primer"
<400> 31
   tttcatgaaa caaatgaatg atgcaag 27
<210> 32
   <211> 27
   <212> DNA
   <213> Artificial Sequence
<220>
   <221> source
   <223> /note="Description of Artificial Sequence: Synthetic primer"
<400> 32
   tttcatgaaa caaatgaatg atgcccg 27
<210> 33
   <211> 27
   <212> DNA
   <213> Artificial Sequence
<220>
   <221> source
   <223> /note="Description of Artificial Sequence: Synthetic primer"
<400> 33
   tttcatgaaa caaatgaatg atgcgcg 27
<210> 34
   <211> 27
   <212> DNA
   <213> Artificial Sequence
<220>
   <221> source
   <223> /note="Description of Artificial Sequence: Synthetic primer"
<400> 34
   tttcatgaaa caaatgaatg atgctcg 27
<210> 35
   <211> 27
   <212> DNA
   <213> Artificial Sequence
<220>
   <221> source
   <223> /note="Description of Artificial Sequence: Synthetic primer"
<400> 35
   tttcatgaaa caaatgaatg atggacg 27
<210> 36
   <211> 27
   <212> DNA
   <213> Artificial Sequence
<220>
   <221> source
   <223> /note="Description of Artificial Sequence: Synthetic primer"
<400> 36
   tttcatgaaa caaatgaatg atgtacg 27
<210> 37
   <211> 27
   <212> DNA
   <213> Artificial Sequence
<220>
   <221> source
   <223> /note="Description of Artificial Sequence: Synthetic primer"
<400> 37
   tttcatgaaa caaatgaatg atgaacg 27
<210> 38
   <211> 23
   <212> DNA
   <213> Artificial Sequence
<220>
   <221> source
   <223> /note="Description of Artificial Sequence: Synthetic primer"
<400> 38
   tttgttgtcc agccaccatg atg 23
<210> 39
   <211> 23
   <212> DNA
   <213> Artificial Sequence
<220>
   <221> source
   <223> /note="Description of Artificial Sequence: Synthetic primer"
<400> 39
   tttgttgtcc agccaccatg aaa 23
<210> 40
   <211> 23
   <212> DNA
   <213> Artificial Sequence
<220>
   <221> source
   <223> /note="Description of Artificial Sequence: Synthetic primer"
<400> 40
   tttgttgtcc agccaccatg aca 23
<210> 41
   <211> 23
   <212> DNA
   <213> Artificial Sequence
<220>
   <221> source
   <223> /note="Description of Artificial Sequence: Synthetic primer"
<400> 41
   tttgttgtcc agccaccatg aga 23
<210> 42
   <211> 23
   <212> DNA
   <213> Artificial Sequence
<220>
   <221> source
   <223> /note="Description of Artificial Sequence: Synthetic primer"
<400> 42
   tttgttgtcc agccaccatg cta 23
<210> 43
   <211> 23
   <212> DNA
   <213> Artificial Sequence
<220>
   <221> source
   <223> /note="Description of Artificial Sequence: Synthetic primer"
<400> 43
   tttgttgtcc agccaccatg gta 23
<210> 44
   <211> 23
   <212> DNA
   <213> Artificial Sequence
<220>
   <221> source
   <223> /note="Description of Artificial Sequence: Synthetic primer"
<400> 44
   tttgttgtcc agccaccatg tta 23
<210> 45
   <211> 23
   <212> DNA
   <213> Artificial Sequence
<220>
   <221> source
   <223> /note="Description of Artificial Sequence: Synthetic primer"
<400> 45
   tttgttgtcc agccaccatc ata 23
<210> 46
   <211> 23
   <212> DNA
   <213> Artificial Sequence
<220>
   <221> source
   <223> /note="Description of Artificial Sequence: Synthetic primer"
<400> 46
   tttgttgtcc agccaccatt ata 23
<210> 47
   <211> 23
   <212> DNA
   <213> Artificial Sequence
<220>
   <221> source
   <223> /note="Description of Artificial Sequence: Synthetic primer"
<400> 47
   tttgttgtcc agccaccata ata 23
<210> 48
   <211> 25
   <212> DNA
   <213> Artificial Sequence
<220>
   <221> source
   <223> /note="Description of Artificial Sequence: Synthetic primer"
<220>
   <221> modified_base
   <222> (23)..(23)
   <223> N6-tert-butyl-benzyl-deoxyadenine
<400> 48
   gttttgttgt ccagccacca tgata 25
<210> 49
   <211> 24
   <212> DNA
   <213> Artificial Sequence
<220>
   <221> source
   <223> /note="Description of Artificial Sequence: Synthetic primer"
<220>
   <221> modified_base
   <222> (22)..(22)
   <223> N6-tert-butyl-benzyl-deoxyadenine
<400> 49
   ttgtgttgtc cagccaccat gata 24
<210> 50
   <211> 30
   <212> DNA
   <213> Artificial Sequence
<220>
   <221> source
   <223> /note="Description of Artificial Sequence: Synthetic primer"
<400> 50
   agtatttcat gaaacaaatg aatgatgcgt 30
<210> 51
   <211> 30
   <212> DNA
   <213> Artificial Sequence
<220>
   <221> source
   <223> /note="Description of Artificial Sequence: Synthetic primer"
<400> 51
   agtatttcat gaaacaaatg aatgatgctt 30
<210> 52
   <211> 30
   <212> DNA
   <213> Artificial Sequence
<220>
   <221> source
   <223> /note="Description of Artificial Sequence: Synthetic primer"
<400> 52
   agtatttcat gaaacaaatg aatgatggat 30
<210> 53
   <211> 30
   <212> DNA
   <213> Artificial Sequence
<220>
   <221> source
   <223> /note="Description of Artificial Sequence: Synthetic primer"
<400> 53
   agtatttcat gaaacaaatg aatgatgtat 30
<210> 54
   <211> 30
   <212> DNA
   <213> Artificial Sequence
<220>
   <221> source
   <223> /note="Description of Artificial Sequence: Synthetic primer"
<400> 54
   agtatttcat gaaacaaatg aatgatgaat 30
<210> 55
   <211> 30
   <212> DNA
   <213> Artificial Sequence
<220>
   <221> source
   <223> /note="Description of Artificial Sequence: Synthetic primer"
<220>
   <221> modified_base
   <222> (29)..(29)
   <223> N6-tert-butyl-benzyl-deoxyadenine
<400> 55
   agtgtttcat gaaacaaatg aatgatgcat 30
<210> 56
   <211> 30
   <212> DNA
   <213> Artificial Sequence
<220>
   <221> source
   <223> /note="Description of Artificial Sequence: Synthetic primer"
<220>
   <221> modified_base
   <222> (28)..(28)
   <223> N4-tert-butyl-benzyl-deoxycytosine
<400> 56
   agtgtttcat gaaacaaatg aatgatgcat 30
<210> 57
   <211> 30
   <212> DNA
   <213> Artificial Sequence
<220>
   <221> source
   <223> /note="Description of Artificial Sequence: Synthetic primer"
<220>
   <221> modified_base
   <222> (28)..(28)
   <223> N4-ethyl-deoxycytosine
<400> 57
   agtatttcat gaaacaaatg aatgatgcgt 30
<210> 58
   <211> 30
   <212> DNA
   <213> Artificial Sequence
<220>
   <221> source
   <223> /note="Description of Artificial Sequence: Synthetic primer"
<220>
   <221> modified_base
   <222> (29)..(29)
   <223> N6-tert-butyl-benzyl-deoxyadenine
<400> 58
   agtatttcat gaaacaaatg aatgattcat 30
<210> 59
   <211> 30
   <212> DNA
   <213> Artificial Sequence
<220>
   <221> source
   <223> /note="Description of Artificial Sequence: Synthetic primer"
<220>
   <221> modified_base
   <222> (28)..(28)
   <223> N4-ethyl-deoxycytosine
<400> 59
   agtatttcat gaaacaaatg aatgatgctt 30
<210> 60
   <211> 30
   <212> DNA
   <213> Artificial Sequence
<220>
   <221> source
   <223> /note="Description of Artificial Sequence: Synthetic primer"
<400> 60
   ataaaaattc tttgtgcaac ctacgtgaat 30
<210> 61
   <211> 30
   <212> DNA
   <213> Artificial Sequence
<220>
   <221> source
   <223> /note="Description of Artificial Sequence: Synthetic primer"
<400> 61
   ataaaaattc tttgtgcaac ctacgtgaaa 30
<210> 62
   <211> 30
   <212> DNA
   <213> Artificial Sequence
<220>
   <221> source
   <223> /note="Description of Artificial Sequence: Synthetic primer"
<400> 62
   ataaaaattc tttgtgcaac ctacgtgaca 30
<210> 63
   <211> 30
   <212> DNA
   <213> Artificial Sequence
<220>
   <221> source
   <223> /note="Description of Artificial Sequence: Synthetic primer"
<400> 63
   ataaaaattc tttgtgcaac ctacgtgaga 30
<210> 64
   <211> 30
   <212> DNA
   <213> Artificial Sequence
<220>
   <221> source
   <223> /note="Description of Artificial Sequence: Synthetic primer"
<400> 64
   ataaaaattc tttgtgcaac ctacgtgata 30
<210> 65
   <211> 30
   <212> DNA
   <213> Artificial Sequence
<220>
   <221> source
   <223> /note="Description of Artificial Sequence: Synthetic primer"
<400> 65
   ataaaaattc tttgtgcaac ctacgtgcaa 30
<210> 66
   <211> 30
   <212> DNA
   <213> Artificial Sequence
<220>
   <221> source
   <223> /note="Description of Artificial Sequence: Synthetic primer"
<400> 66
   ataaaaattc tttgtgcaac ctacgtggaa 30
<210> 67
   <211> 30
   <212> DNA
   <213> Artificial Sequence
<220>
   <221> source
   <223> /note="Description of Artificial Sequence: Synthetic primer"
<400> 67
   ataaaaattc tttgtgcaac ctacgtgtaa 30
<210> 68
   <211> 30
   <212> DNA
   <213> Artificial Sequence
<220>
   <221> source
   <223> /note="Description of Artificial Sequence: Synthetic primer"
<400> 68
   ataaaaattc tttgtgcaac ctacgtcaaa 30
<210> 69
   <211> 30
   <212> DNA
   <213> Artificial Sequence
<220>
   <221> source
   <223> /note="Description of Artificial Sequence: Synthetic primer"
<400> 69
   ataaaaattc tttgtgcaac ctacgttaaa 30
<210> 70
   <211> 30
   <212> DNA
   <213> Artificial Sequence
<220>
   <221> source
   <223> /note="Description of Artificial Sequence: Synthetic primer"
<400> 70
   ataaaaattc tttgtgcaac ctacgtaaaa 30
<210> 71
   <211> 30
   <212> DNA
   <213> Artificial Sequence
<220>
   <221> source
   <223> /note="Description of Artificial Sequence: Synthetic primer"
<400> 71
   atagaaattc tttgtgcaac ctacgtgaaa 30
<210> 72
   <211> 30
   <212> DNA
   <213> Artificial Sequence
<220>
   <221> source
   <223> /note="Description of Artificial Sequence: Synthetic primer"
<220>
   <221> modified_base
   <222> (30)..(30)
   <223> N6-tert-butyl-benzyl-deoxyadenine
<400> 72
   atgaaaattc tttgtgcaac ctacgtgaaa 30
<210> 73
   <211> 30
   <212> DNA
   <213> Artificial Sequence
<220>
   <221> source
   <223> /note="Description of Artificial Sequence: Synthetic primer"
<220>
   <221> modified_base
   <222> (29)..(29)
   <223> N6-tert-butyl-benzyl-deoxyadenine
<400> 73
   atgaaaattc tttgtgcaac ctacgtgaaa 30
<210> 74
   <211> 30
   <212> DNA
   <213> Artificial Sequence
<220>
   <221> source
   <223> /note="Description of Artificial Sequence: Synthetic primer"
<220>
   <221> modified_base
   <222> (28)..(28)
   <223> N6-tert-butyl-benzyl-deoxyadenine
<400> 74
   atgaaaattc tttgtgcaac ctacgtgaaa 30
<210> 75
   <211> 30
   <212> DNA
   <213> Artificial Sequence
<220>
   <221> source
   <223> /note="Description of Artificial Sequence: Synthetic primer"
<220>
   <221> modified_base
   <222> (29)..(29)
   <223> N4-tert-butyl-benzyl-deoxycytosine
<400> 75
   ataaaaattc tttgtgcaac ctacgtgaca 30
<210> 76
   <211> 30
   <212> DNA
   <213> Artificial Sequence
<220>
   <221> source
   <223> /note="Description of Artificial Sequence: Synthetic primer"
<220>
   <221> modified_base
   <222> (28)..(28)
   <223> N4-tert-butyl-benzyl-deoxycytosine
<400> 76
   ataaaaattc tttgtgcaac ctacgtgcaa 30
<210> 77
   <211> 30
   <212> DNA
   <213> Artificial Sequence
<220>
   <221> source
   <223> /note="Description of Artificial Sequence: Synthetic primer"
<220>
   <221> modified_base
   <222> (29)..(29)
   <223> N4-methyl-deoxycytosine
<400> 77
   ataaaaattc tttgtgcaac ctacgtgaca 30
<210> 78
   <211> 30
   <212> DNA
   <213> Artificial Sequence
<220>
   <221> source
   <223> /note="Description of Artificial Sequence: Synthetic primer"
<220>
   <221> modified_base
   <222> (30)..(30)
   <223> N6-tert-butyl-benzyl-deoxyadenine
<400> 78
   ataaaaattc tttgtgcaac ctacgggaaa 30
<210> 79
   <211> 30
   <212> DNA
   <213> Artificial Sequence
<220>
   <221> source
   <223> /note="Description of Artificial Sequence: Synthetic primer"
<220>
   <221> modified_base
   <222> (27)..(27)
   <223> N4-tert-butyl-benzyl-deoxycytosine
<400> 79
   ataaaaattc tttgtgcaac ctacgtcaaa 30
<210> 80
   <211> 30
   <212> DNA
   <213> Artificial Sequence
<220>
   <221> source
   <223> /note="Description of Artificial Sequence: Synthetic primer"
<220>
   <221> modified_base
   <222> (29)..(29)
   <223> N6-tert-butyl-benzyl-deoxyadenine
<400> 80
   ataaaaattc tttgtgcaac ctacgggaaa 30
<210> 81
   <211> 30
   <212> DNA
   <213> Artificial Sequence
<220>
   <221> source
   <223> /note="Description of Artificial Sequence: Synthetic primer"
<220>
   <221> modified_base
   <222> (27)..(27)
   <223> N4-methyl-deoxycytosine
<400> 81
   ataaaaattc tttgtgcaac ctacgtcaaa 30
<210> 82
   <211> 30
   <212> DNA
   <213> Artificial Sequence
<220>
   <221> source
   <223> /note="Description of Artificial Sequence: Synthetic primer"
<220>
   <221> modified_base
   <222> (27)..(27)
   <223> N4-methyl-deoxycytosine
<220>
   <221> modified_base
   <222> (30)..(30)
   <223> N6-tert-butyl-benzyl-deoxyadenine
<400> 82
   ataaaaattc tttgtgcaac ctacgtcaaa 30
<210> 83
   <211> 27
   <212> DNA
   <213> Artificial Sequence
<220>
   <221> source
   <223> /note="Description of Artificial Sequence: Synthetic primer"
<400> 83
   caatttctac acgagatcct ctctctg 27
<210> 84
   <211> 27
   <212> DNA
   <213> Artificial Sequence
<220>
   <221> source
   <223> /note="Description of Artificial Sequence: Synthetic primer"
<400> 84
   caatttctac acgagatcct ctctcta 27
<210> 85
   <211> 27
   <212> DNA
   <213> Artificial Sequence
<220>
   <221> source
   <223> /note="Description of Artificial Sequence: Synthetic primer"
<400> 85
   caatttctac acgagatcct ctctcaa 27
<210> 86
   <211> 27
   <212> DNA
   <213> Artificial Sequence
<220>
   <221> source
   <223> /note="Description of Artificial Sequence: Synthetic primer"
<400> 86
   caatttctac acgagatcct ctctcca 27
<210> 87
   <211> 27
   <212> DNA
   <213> Artificial Sequence
<220>
   <221> source
   <223> /note="Description of Artificial Sequence: Synthetic primer"
<400> 87
   caatttctac acgagatcct ctctcga 27
<210> 88
   <211> 27
   <212> DNA
   <213> Artificial Sequence
<220>
   <221> source
   <223> /note="Description of Artificial Sequence: Synthetic primer"
<400> 88
   caatttctac acgagatcct ctctgta 27
<210> 89
   <211> 27
   <212> DNA
   <213> Artificial Sequence
<220>
   <221> source
   <223> /note="Description of Artificial Sequence: Synthetic primer"
<400> 89
   caatttctac acgagatcct ctcttta 27
<210> 90
   <211> 27
   <212> DNA
   <213> Artificial Sequence
<220>
   <221> source
   <223> /note="Description of Artificial Sequence: Synthetic primer"
<400> 90
   caatttctac acgagatcct ctctata 27
<210> 91
   <211> 27
   <212> DNA
   <213> Artificial Sequence
<220>
   <221> source
   <223> /note="Description of Artificial Sequence: Synthetic primer"
<400> 91
   caatttctac acgagatcct ctcacta 27
<210> 92
   <211> 27
   <212> DNA
   <213> Artificial Sequence
<220>
   <221> source
   <223> /note="Description of Artificial Sequence: Synthetic primer"
<400> 92
   caatttctac acgagatcct ctcccta 27
<210> 93
   <211> 27
   <212> DNA
   <213> Artificial Sequence
<220>
   <221> source
   <223> /note="Description of Artificial Sequence: Synthetic primer"
<400> 93
   caatttctac acgagatcct ctcgcta 27
<210> 94
   <211> 27
   <212> DNA
   <213> Artificial Sequence
<220>
   <221> source
   <223> /note="Description of Artificial Sequence: Synthetic primer"
<400> 94
   cagtttctac acgagatcct ctctcta 27
<210> 95
   <211> 31
   <212> DNA
   <213> Artificial Sequence
<220>
   <221> source
   <223> /note="Description of Artificial Sequence: Synthetic primer"
<220>
   <221> modified_base
   <222> (31)..(31)
   <223> N6-tert-butyl-benzyl-deoxyadenine
<400> 95
   gaagcaattt ctacacgaga tcctctctct a 31
<210> 96
   <211> 31
   <212> DNA
   <213> Artificial Sequence
<220>
   <221> source
   <223> /note="Description of Artificial Sequence: Synthetic primer"
<220>
   <221> modified_base
   <222> (29)..(29)
   <223> N4-tert-butyl-benzyl-deoxycytosine
<400> 96
   gaagcaattt ctacacgaga tcctctctct a 31
<210> 97
   <211> 27
   <212> DNA
   <213> Artificial Sequence
<220>
   <221> source
   <223> /note="Description of Artificial Sequence: Synthetic primer"
<220>
   <221> modified_base
   <222> (25)..(25)
   <223> N4-tert-butyl-benzyl-deoxycytosine
<400> 97
   cagtttctac acgagatcct ctctcta 27
<210> 98
   <211> 25
   <212> DNA
   <213> Artificial Sequence
<220>
   <221> source
   <223> /note="Description of Artificial Sequence: Synthetic primer"
<400> 98
   gagatcctct ctctgaaatc actga 25
<210> 99
   <211> 25
   <212> DNA
   <213> Artificial Sequence
<220>
   <221> source
   <223> /note="Description of Artificial Sequence: Synthetic primer"
<400> 99
   gagatcctct ctctgaaatc actgc 25
<210> 100
   <211> 25
   <212> DNA
   <213> Artificial Sequence
<220>
   <221> source
   <223> /note="Description of Artificial Sequence: Synthetic primer"
<400> 100
   gagatcctct ctctgaaatc actcc 25
<210> 101
   <211> 25
   <212> DNA
   <213> Artificial Sequence
<220>
   <221> source
   <223> /note="Description of Artificial Sequence: Synthetic primer"
<400> 101
   gagatcctct ctctgaaatc acttc 25
<210> 102
   <211> 25
   <212> DNA
   <213> Artificial Sequence
<220>
   <221> source
   <223> /note="Description of Artificial Sequence: Synthetic primer"
<400> 102
   gagatcctct ctctgaaatc actac 25
<210> 103
   <211> 25
   <212> DNA
   <213> Artificial Sequence
<220>
   <221> source
   <223> /note="Description of Artificial Sequence: Synthetic primer"
<400> 103
   gagatcctct ctctgaaatc acagc 25
<210> 104
   <211> 25
   <212> DNA
   <213> Artificial Sequence
<220>
   <221> source
   <223> /note="Description of Artificial Sequence: Synthetic primer"
<400> 104
   gagatcctct ctctgaaatc accgc 25
<210> 105
   <211> 25
   <212> DNA
   <213> Artificial Sequence
<220>
   <221> source
   <223> /note="Description of Artificial Sequence: Synthetic primer"
<400> 105
   gagatcctct ctctgaaatc acggc 25
<210> 106
   <211> 25
   <212> DNA
   <213> Artificial Sequence
<220>
   <221> source
   <223> /note="Description of Artificial Sequence: Synthetic primer"
<400> 106
   gagatcctct ctctgaaatc agtgc 25
<210> 107
   <211> 25
   <212> DNA
   <213> Artificial Sequence
<220>
   <221> source
   <223> /note="Description of Artificial Sequence: Synthetic primer"
<400> 107
   gagatcctct ctctgaaatc attgc 25
<210> 108
   <211> 25
   <212> DNA
   <213> Artificial Sequence
<220>
   <221> source
   <223> /note="Description of Artificial Sequence: Synthetic primer"
<400> 108
   gagatcctct ctctgaaatc aatgc 25
<210> 109
   <211> 25
   <212> DNA
   <213> Artificial Sequence
<220>
   <221> source
   <223> /note="Description of Artificial Sequence: Synthetic primer"
<400> 109
   gggatcctct ctctgaaatc actgc 25
<210> 110
   <211> 25
   <212> DNA
   <213> Artificial Sequence
<220>
   <221> source
   <223> /note="Description of Artificial Sequence: Synthetic primer"
<220>
   <221> modified_base
   <222> (22)..(22)
   <223> N4-tert-butyl-benzyl-deoxycytosine
<400> 110
   gggatcctct ctctgaaatc actgc 25
<210> 111
   <211> 25
   <212> DNA
   <213> Artificial Sequence
<220>
   <221> source
   <223> /note="Description of Artificial Sequence: Synthetic primer"
<220>
   <221> modified_base
   <222> (25)..(25)
   <223> N4-tert-butyl-benzyl-deoxycytosine
<400> 111
   gggatcctct ctctgaaatc actgc 25
<210> 112
   <211> 25
   <212> DNA
   <213> Artificial Sequence
<220>
   <221> source
   <223> /note="Description of Artificial Sequence: Synthetic primer"
<220>
   <221> modified_base
   <222> (25)..(25)
   <223> N4-tert-butyl-benzyl-deoxycytosine
<400> 112
   gagatcctct ctctgaaatc gctgc 25
<210> 113
   <211> 25
   <212> DNA
   <213> Artificial Sequence
<220>
   <221> source
   <223> /note="Description of Artificial Sequence: Synthetic primer"
<220>
   <221> modified_base
   <222> (25)..(25)
   <223> N4-tert-butyl-benzyl-deoxycytosine
<400> 113
   gagatcctct ctctgaaatc attgc 25
<210> 114
   <211> 25
   <212> DNA
   <213> Artificial Sequence
<220>
   <221> source
   <223> /note="Description of Artificial Sequence: Synthetic primer"
<220>
   <221> modified_base
   <222> (24)..(24)
   <223> N4-tert-butyl-benzyl-deoxycytosine
<400> 114
   gagatcctct ctctgaaatc actcc 25
<210> 115
   <211> 25
   <212> DNA
   <213> Artificial Sequence
<220>
   <221> source
   <223> /note="Description of Artificial Sequence: Synthetic primer"
<220>
   <221> modified_base
   <222> (21)..(21)
   <223> N6-tert-butyl-benzyl-deoxyadenine
<220>
   <221> modified_base
   <222> (24)..(24)
   <223> Gclamp
<400> 115
   gagatcctct ctctgaaatc actcc 25
<210> 116
   <211> 25
   <212> DNA
   <213> Artificial Sequence
<220>
   <221> source
   <223> /note="Description of Artificial Sequence: Synthetic primer"
<220>
   <221> modified_base
   <222> (23)..(23)
   <223> N4-tert-butyl-benzyl-deoxycytosine
<400> 116
   gagatcctct ctctgaaatc accgc 25
<210> 117
   <211> 25
   <212> DNA
   <213> Artificial Sequence
<220>
   <221> source
   <223> /note="Description of Artificial Sequence: Synthetic primer"
<220>
   <221> modified_base
   <222> (24)..(24)
   <223> N6-tert-butyl-benzyl-deoxyadenine
<400> 117
   gagatcctct ctctgaaatc actac 25
<210> 118
   <211> 25
   <212> DNA
   <213> Artificial Sequence
<220>
   <221> source
   <223> /note="Description of Artificial Sequence: Synthetic primer"
<220>
   <221> modified_base
   <222> (23)..(23)
   <223> N4-ethyl-deoxycytosine
<400> 118
   gagatcctct ctctgaaatc gccgc 25
<210> 119
   <211> 25
   <212> DNA
   <213> Artificial Sequence
<220>
   <221> source
   <223> /note="Description of Artificial Sequence: Synthetic primer"
<220>
   <221> modified_base
   <222> (25)..(25)
   <223> N4-tert-butyl-benzyl-deoxycytosine
<400> 119
   gagatcctct ctctgaaatc accgc 25
<210> 120
   <211> 25
   <212> DNA
   <213> Artificial Sequence
<220>
   <221> source
   <223> /note="Description of Artificial Sequence: Synthetic primer"
<220>
   <221> modified_base
   <222> (25)..(25)
   <223> N4-tert-butyl-benzyl-deoxycytosine
<400> 120
   gagatcctct ctctgaaatc acggc 25
<210> 121
   <211> 25
   <212> DNA
   <213> Artificial Sequence
<220>
   <221> source
   <223> /note="Description of Artificial Sequence: Synthetic primer"
<220>
   <221> modified_base
   <222> (22)..(22)
   <223> N4-ethyl-deoxycytosine
<220>
   <221> modified_base
   <222> (24)..(24)
   <223> N4-ethyl-deoxycytosine
<400> 121
   gagatcctct ctctgaaatc actcc 25
<210> 122
   <211> 25
   <212> DNA
   <213> Artificial Sequence
<220>
   <221> source
   <223> /note="Description of Artificial Sequence: Synthetic primer"
<220>
   <221> modified_base
   <222> (22)..(22)
   <223> N4-ethyl-deoxycytosine
<400> 122
   gagatcctct ctctgaaatc acggc 25
<210> 123
   <211> 25
   <212> DNA
   <213> Artificial Sequence
<220>
   <221> source
   <223> /note="Description of Artificial Sequence: Synthetic primer"
<220>
   <221> modified_base
   <222> (22)..(22)
   <223> N4-tert-butyl-benzyl-deoxycytosine
<400> 123
   gagatcctct ctctgaaatc accgc 25
<210> 124
   <211> 25
   <212> DNA
   <213> Artificial Sequence
<220>
   <221> source
   <223> /note="Description of Artificial Sequence: Synthetic primer"
<220>
   <221> modified_base
   <222> (23)..(23)
   <223> N6-tert-butyl-benzyl-deoxyadenine
<400> 124
   gagatcctct ctctgaaatc acagc 25
<210> 125
   <211> 25
   <212> DNA
   <213> Artificial Sequence
<220>
   <221> source
   <223> /note="Description of Artificial Sequence: Synthetic primer"
<400> 125
   gagatcctct ctctgaaatc actga 25
<210> 126
   <211> 25
   <212> DNA
   <213> Artificial Sequence
<220>
   <221> source
   <223> /note="Description of Artificial Sequence: Synthetic primer"
<400> 126
   gagatcctct ctctgaaatc actgg 25
<210> 127
   <211> 25
   <212> DNA
   <213> Artificial Sequence
<220>
   <221> source
   <223> /note="Description of Artificial Sequence: Synthetic primer"
<400> 127
   gagatcctct ctctgaaatc actcg 25
<210> 128
   <211> 25
   <212> DNA
   <213> Artificial Sequence
<220>
   <221> source
   <223> /note="Description of Artificial Sequence: Synthetic primer"
<400> 128
   gagatcctct ctctgaaatc actag 25
<210> 129
   <211> 25
   <212> DNA
   <213> Artificial Sequence
<220>
   <221> source
   <223> /note="Description of Artificial Sequence: Synthetic primer"
<400> 129
   gagatcctct ctctgaaatc acttg 25
<210> 130
   <211> 25
   <212> DNA
   <213> Artificial Sequence
<220>
   <221> source
   <223> /note="Description of Artificial Sequence: Synthetic primer"
<400> 130
   gagatcctct ctctgaaatc acagg 25
<210> 131
   <211> 25
   <212> DNA
   <213> Artificial Sequence
<220>
   <221> source
   <223> /note="Description of Artificial Sequence: Synthetic primer"
<400> 131
   gagatcctct ctctgaaatc accgg 25
<210> 132
   <211> 25
   <212> DNA
   <213> Artificial Sequence
<220>
   <221> source
   <223> /note="Description of Artificial Sequence: Synthetic primer"
<400> 132
   gagatcctct ctctgaaatc acggg 25
<210> 133
   <211> 25
   <212> DNA
   <213> Artificial Sequence
<220>
   <221> source
   <223> /note="Description of Artificial Sequence: Synthetic primer"
<400> 133
   gagatcctct ctctgaaatc agtgg 25
<210> 134
   <211> 25
   <212> DNA
   <213> Artificial Sequence
<220>
   <221> source
   <223> /note="Description of Artificial Sequence: Synthetic primer"
<400> 134
   gagatcctct ctctgaaatc aatgg 25
<210> 135
   <211> 25
   <212> DNA
   <213> Artificial Sequence
<220>
   <221> source
   <223> /note="Description of Artificial Sequence: Synthetic primer"
<400> 135
   gagatcctct ctctgaaatc attgg 25
<210> 136
   <211> 25
   <212> DNA
   <213> Artificial Sequence
<220>
   <221> source
   <223> /note="Description of Artificial Sequence: Synthetic primer"
<400> 136
   gggatcctct ctctgaaatc actgg 25
<210> 137
   <211> 25
   <212> DNA
   <213> Artificial Sequence
<220>
   <221> source
   <223> /note="Description of Artificial Sequence: Synthetic primer"
<220>
   <221> modified_base
   <222> (22)..(22)
   <223> N4-tert-butyl-benzyl-deoxycytosine
<400> 137
   gggatcctct ctctgaaatc actgg 25
<210> 138
   <211> 25
   <212> DNA
   <213> Artificial Sequence
<220>
   <221> source
   <223> /note="Description of Artificial Sequence: Synthetic primer"
<220>
   <221> modified_base
   <222> (24)..(24)
   <223> N4-tert-butyl-benzyl-deoxycytosine
<400> 138
   gagatcctct ctctgaaatc actcg 25
<210> 139
   <211> 25
   <212> DNA
   <213> Artificial Sequence
<220>
   <221> source
   <223> /note="Description of Artificial Sequence: Synthetic primer"
<220>
   <221> modified_base
   <222> (21)..(21)
   <223> N6-tert-butyl-benzyl-deoxyadenine
<220>
   <221> modified_base
   <222> (24)..(24)
   <223> N4-ethyl-deoxycytosine
<400> 139
   gagatcctct ctctgaaatc actcg 25
<210> 140
   <211> 25
   <212> DNA
   <213> Artificial Sequence
<220>
   <221> source
   <223> /note="Description of Artificial Sequence: Synthetic primer"
<220>
   <221> modified_base
   <222> (21)..(21)
   <223> N6-tert-butyl-benzyl-deoxyadenine
<400> 140
   gagatcctct ctctgaaatc acttg 25
<210> 141
   <211> 25
   <212> DNA
   <213> Artificial Sequence
<220>
   <221> source
   <223> /note="Description of Artificial Sequence: Synthetic primer"
<220>
   <221> modified_base
   <222> (24)..(24)
   <223> N6-tert-butyl-benzyl-deoxyadenine
<400> 141
   gagatcctct ctctgaaatc actag 25
<210> 142
   <211> 25
   <212> DNA
   <213> Artificial Sequence
<220>
   <221> source
   <223> /note="Description of Artificial Sequence: Synthetic primer"
<220>
   <221> modified_base
   <222> (24)..(24)
   <223> N4-ethyl-deoxycytosine
<400> 142
   gagatcctct ctctgaaatc actcg 25
<210> 143
   <211> 25
   <212> DNA
   <213> Artificial Sequence
<220>
   <221> source
   <223> /note="Description of Artificial Sequence: Synthetic primer"
<220>
   <221> modified_base
   <222> (22)..(22)
   <223> N6-tert-butyl-benzyl-deoxyadenine
<400> 143
   gagatcctct ctctgaaatc aatgg 25
<210> 144
   <211> 31
   <212> DNA
   <213> Artificial Sequence
<220>
   <221> source
   <223> /note="Description of Artificial Sequence: Synthetic primer"
<220>
   <221> modified_base
   <222> (21)..(21)
   <223> Inosine
<220>
   <221> modified_base
   <222> (28)..(28)
   <223> N4-tert-butyl-benzyl-deoxycytosine
<400> 144
   ctatacgaga tcctctctct naaatcactg g 31
<210> 145
   <211> 24
   <212> DNA
   <213> Artificial Sequence
<220>
   <221> source
   <223> /note="Description of Artificial Sequence: Synthetic primer"
<400> 145
   agatcctctc tctgaaatca ctag 24
<210> 146
   <211> 24
   <212> DNA
   <213> Artificial Sequence
<220>
   <221> source
   <223> /note="Description of Artificial Sequence: Synthetic primer"
<400> 146
   agatcctctc tctgaaatca cggg 24
<210> 147
   <211> 26
   <212> DNA
   <213> Artificial Sequence
<220>
   <221> source
   <223> /note="Description of Artificial Sequence: Synthetic primer"
<400> 147
   acgagatcct ctctctgaaa tcactg 26
<210> 148
   <211> 26
   <212> DNA
   <213> Artificial Sequence
<220>
   <221> source
   <223> /note="Description of Artificial Sequence: Synthetic primer"
<400> 148
   acgagatcct ctctctgaaa tcacta 26
<210> 149
   <211> 26
   <212> DNA
   <213> Artificial Sequence
<220>
   <221> source
   <223> /note="Description of Artificial Sequence: Synthetic primer"
<400> 149
   acgagatcct ctctctgaaa tcacaa 26
<210> 150
   <211> 26
   <212> DNA
   <213> Artificial Sequence
<220>
   <221> source
   <223> /note="Description of Artificial Sequence: Synthetic primer"
<400> 150
   acgagatcct ctctctgaaa tcacca 26
<210> 151
   <211> 26
   <212> DNA
   <213> Artificial Sequence
<220>
   <221> source
   <223> /note="Description of Artificial Sequence: Synthetic primer"
<400> 151
   acgagatcct ctctctgaaa tcacga 26
<210> 152
   <211> 26
   <212> DNA
   <213> Artificial Sequence
<220>
   <221> source
   <223> /note="Description of Artificial Sequence: Synthetic primer"
<400> 152
   acgagatcct ctctctgaaa tcagta 26
<210> 153
   <211> 26
   <212> DNA
   <213> Artificial Sequence
<220>
   <221> source
   <223> /note="Description of Artificial Sequence: Synthetic primer"
<400> 153
   acgagatcct ctctctgaaa tcaata 26
<210> 154
   <211> 26
   <212> DNA
   <213> Artificial Sequence
<220>
   <221> source
   <223> /note="Description of Artificial Sequence: Synthetic primer"
<400> 154
   acgagatcct ctctctgaaa tcatta 26
<210> 155
   <211> 26
   <212> DNA
   <213> Artificial Sequence
<220>
   <221> source
   <223> /note="Description of Artificial Sequence: Synthetic primer"
<400> 155
   acgagatcct ctctctgaaa tcccta 26
<210> 156
   <211> 26
   <212> DNA
   <213> Artificial Sequence
<220>
   <221> source
   <223> /note="Description of Artificial Sequence: Synthetic primer"
<400> 156
   acgagatcct ctctctgaaa tcgcta 26
<210> 157
   <211> 26
   <212> DNA
   <213> Artificial Sequence
<220>
   <221> source
   <223> /note="Description of Artificial Sequence: Synthetic primer"
<400> 157
   acgagatcct ctctctgaaa tctcta 26
<210> 158
   <211> 26
   <212> DNA
   <213> Artificial Sequence
<220>
   <221> source
   <223> /note="Description of Artificial Sequence: Synthetic primer"
<400> 158
   aggagatcct ctctctgaaa tcacta 26
<210> 159
   <211> 26
   <212> DNA
   <213> Artificial Sequence
<220>
   <221> source
   <223> /note="Description of Artificial Sequence: Synthetic primer"
<220>
   <221> modified_base
   <222> (26)..(26)
   <223> N6-tert-butyl-benzyl-deoxyadenine
<400> 159
   aggagatcct ctctctgaaa tcacta 26
<210> 160
   <211> 26
   <212> DNA
   <213> Artificial Sequence
<220>
   <221> source
   <223> /note="Description of Artificial Sequence: Synthetic primer"
<220>
   <221> modified_base
   <222> (24)..(24)
   <223> N4-ethyl-deoxycytosine
<400> 160
   aggagatcct ctctctgaaa tcacta 26
<210> 161
   <211> 26
   <212> DNA
   <213> Artificial Sequence
<220>
   <221> source
   <223> /note="Description of Artificial Sequence: Synthetic primer"
<220>
   <221> modified_base
   <222> (23)..(23)
   <223> N6-tert-butyl-benzyl-deoxyadenine
<400> 161
   aggagatcct ctctctgaaa tcacta 26
<210> 162
   <211> 26
   <212> DNA
   <213> Artificial Sequence
<220>
   <221> source
   <223> /note="Description of Artificial Sequence: Synthetic primer"
<220>
   <221> modified_base
   <222> (24)..(24)
   <223> N6-tert-butyl-benzyl-deoxyadenine
<400> 162
   acgagatcct ctctctgaaa tcaata 26
<210> 163
   <211> 26
   <212> DNA
   <213> Artificial Sequence
<220>
   <221> source
   <223> /note="Description of Artificial Sequence: Synthetic primer"
<220>
   <221> modified_base
   <222> (25)..(25)
   <223> N6-tert-butyl-benzyl-deoxyadenine
<400> 163
   acgagatcct ctctctgaaa tcacaa 26
<210> 164
   <211> 26
   <212> DNA
   <213> Artificial Sequence
<220>
   <221> source
   <223> /note="Description of Artificial Sequence: Synthetic primer"
<220>
   <221> modified_base
   <222> (22)..(22)
   <223> N4-methyl-deoxycytosine
<220>
   <221> modified_base
   <222> (24)..(24)
   <223> N6-tert-butyl-benzyl-deoxyadenine
<400> 164
   acgagatcct ctctctgaaa tcaata 26
<210> 165
   <211> 26
   <212> DNA
   <213> Artificial Sequence
<220>
   <221> source
   <223> /note="Description of Artificial Sequence: Synthetic primer"
<220>
   <221> modified_base
   <222> (25)..(25)
   <223> N4-tert-butyl-benzyl-deoxycytosine
<400> 165
   acgagatcct ctctctgaaa tcacca 26
<210> 166
   <211> 26
   <212> DNA
   <213> Artificial Sequence
<220>
   <221> source
   <223> /note="Description of Artificial Sequence: Synthetic primer"
<220>
   <221> modified_base
   <222> (23)..(23)
   <223> N4-tert-butyl-benzyl-deoxycytosine
<400> 166
   acgagatcct ctctctgaaa tcccta 26
<210> 167
   <211> 26
   <212> DNA
   <213> Artificial Sequence
<220>
   <221> source
   <223> /note="Description of Artificial Sequence: Synthetic primer"
<400> 167
   agatcctctc tctgaaatca ctgagc 26
<210> 168
   <211> 26
   <212> DNA
   <213> Artificial Sequence
<220>
   <221> source
   <223> /note="Description of Artificial Sequence: Synthetic primer"
<400> 168
   agatcctctc tctgaaatca ctgaga 26
<210> 169
   <211> 26
   <212> DNA
   <213> Artificial Sequence
<220>
   <221> source
   <223> /note="Description of Artificial Sequence: Synthetic primer"
<400> 169
   agatcctctc tctgaaatca ctgaca 26
<210> 170
   <211> 26
   <212> DNA
   <213> Artificial Sequence
<220>
   <221> source
   <223> /note="Description of Artificial Sequence: Synthetic primer"
<400> 170
   agatcctctc tctgaaatca ctgaaa 26
<210> 171
   <211> 26
   <212> DNA
   <213> Artificial Sequence
<220>
   <221> source
   <223> /note="Description of Artificial Sequence: Synthetic primer"
<400> 171
   agatcctctc tctgaaatca ctgata 26
<210> 172
   <211> 26
   <212> DNA
   <213> Artificial Sequence
<220>
   <221> source
   <223> /note="Description of Artificial Sequence: Synthetic primer"
<400> 172
   agatcctctc tctgaaatca ctgcga 26
<210> 173
   <211> 26
   <212> DNA
   <213> Artificial Sequence
<220>
   <221> source
   <223> /note="Description of Artificial Sequence: Synthetic primer"
<400> 173
   agatcctctc tctgaaatca ctgtga 26
<210> 174
   <211> 26
   <212> DNA
   <213> Artificial Sequence
<220>
   <221> source
   <223> /note="Description of Artificial Sequence: Synthetic primer"
<400> 174
   agatcctctc tctgaaatca ctggga 26
<210> 175
   <211> 26
   <212> DNA
   <213> Artificial Sequence
<220>
   <221> source
   <223> /note="Description of Artificial Sequence: Synthetic primer"
<400> 175
   agatcctctc tctgaaatca ctcaga 26
<210> 176
   <211> 26
   <212> DNA
   <213> Artificial Sequence
<220>
   <221> source
   <223> /note="Description of Artificial Sequence: Synthetic primer"
<400> 176
   agatcctctc tctgaaatca ctaaga 26
<210> 177
   <211> 26
   <212> DNA
   <213> Artificial Sequence
<220>
   <221> source
   <223> /note="Description of Artificial Sequence: Synthetic primer"
<400> 177
   agatcctctc tctgaaatca cttaga 26
<210> 178
   <211> 26
   <212> DNA
   <213> Artificial Sequence
<220>
   <221> source
   <223> /note="Description of Artificial Sequence: Synthetic primer"
<400> 178
   aggtcctctc tctgaaatca ctgaga 26
<210> 179
   <211> 27
   <212> DNA
   <213> Artificial Sequence
<220>
   <221> source
   <223> /note="Description of Artificial Sequence: Synthetic primer"
<220>
   <221> modified_base
   <222> (27)..(27)
   <223> N6-tert-butyl-benzyl-deoxyadenine
<400> 179
   gaggtcctct ctctgaaatc actgaga 27
<210> 180
   <211> 27
   <212> DNA
   <213> Artificial Sequence
<220>
   <221> source
   <223> /note="Description of Artificial Sequence: Synthetic primer"
<220>
   <221> modified_base
   <222> (25)..(25)
   <223> N6-tert-butyl-benzyl-deoxyadenine
<400> 180
   gaggtcctct ctctgaaatc actgaga 27
<210> 181
   <211> 27
   <212> DNA
   <213> Artificial Sequence
<220>
   <221> source
   <223> /note="Description of Artificial Sequence: Synthetic primer"
<400> 181
   gagatcctct ctctgaaatc actgaaa 27
<210> 182
   <211> 27
   <212> DNA
   <213> Artificial Sequence
<220>
   <221> source
   <223> /note="Description of Artificial Sequence: Synthetic primer"
<400> 182
   gagatcctct ctctgaaatc actggga 27
<210> 183
   <211> 27
   <212> DNA
   <213> Artificial Sequence
<220>
   <221> source
   <223> /note="Description of Artificial Sequence: Synthetic primer"
<400> 183
   gagatcctct ctctgaaatc actaaga 27
<210> 184
   <211> 24
   <212> DNA
   <213> Artificial Sequence
<220>
   <221> source
   <223> /note="Description of Artificial Sequence: Synthetic primer"
<400> 184
   atcctctctc tgaaatcact gagc 24
<210> 185
   <211> 24
   <212> DNA
   <213> Artificial Sequence
<220>
   <221> source
   <223> /note="Description of Artificial Sequence: Synthetic primer"
<400> 185
   atcctctctc tgaaatcact gagg 24
<210> 186
   <211> 24
   <212> DNA
   <213> Artificial Sequence
<220>
   <221> source
   <223> /note="Description of Artificial Sequence: Synthetic primer"
<400> 186
   atcctctctc tgaaatcact gaag 24
<210> 187
   <211> 24
   <212> DNA
   <213> Artificial Sequence
<220>
   <221> source
   <223> /note="Description of Artificial Sequence: Synthetic primer"
<400> 187
   atcctctctc tgaaatcact gacg 24
<210> 188
   <211> 24
   <212> DNA
   <213> Artificial Sequence
<220>
   <221> source
   <223> /note="Description of Artificial Sequence: Synthetic primer"
<400> 188
   atcctctctc tgaaatcact gatg 24
<210> 189
   <211> 24
   <212> DNA
   <213> Artificial Sequence
<220>
   <221> source
   <223> /note="Description of Artificial Sequence: Synthetic primer"
<400> 189
   atcctctctc tgaaatcact gcgg 24
<210> 190
   <211> 24
   <212> DNA
   <213> Artificial Sequence
<220>
   <221> source
   <223> /note="Description of Artificial Sequence: Synthetic primer"
<400> 190
   atcctctctc tgaaatcact gggg 24
<210> 191
   <211> 24
   <212> DNA
   <213> Artificial Sequence
<220>
   <221> source
   <223> /note="Description of Artificial Sequence: Synthetic primer"
<400> 191
   atcctctctc tgaaatcact gtgg 24
<210> 192
   <211> 24
   <212> DNA
   <213> Artificial Sequence
<220>
   <221> source
   <223> /note="Description of Artificial Sequence: Synthetic primer"
<400> 192
   atcctctctc tgaaatcact aagg 24
<210> 193
   <211> 24
   <212> DNA
   <213> Artificial Sequence
<220>
   <221> source
   <223> /note="Description of Artificial Sequence: Synthetic primer"
<400> 193
   atcctctctc tgaaatcact cagg 24
<210> 194
   <211> 24
   <212> DNA
   <213> Artificial Sequence
<220>
   <221> source
   <223> /note="Description of Artificial Sequence: Synthetic primer"
<400> 194
   atcctctctc tgaaatcact tagg 24
<210> 195
   <211> 24
   <212> DNA
   <213> Artificial Sequence
<220>
   <221> source
   <223> /note="Description of Artificial Sequence: Synthetic primer"
<400> 195
   tcctctctct gaaatcactg agca 24
<210> 196
   <211> 24
   <212> DNA
   <213> Artificial Sequence
<220>
   <221> source
   <223> /note="Description of Artificial Sequence: Synthetic primer"
<400> 196
   tcctctctct gaaatcactg agca 24
<210> 197
   <211> 24
   <212> DNA
   <213> Artificial Sequence
<220>
   <221> source
   <223> /note="Description of Artificial Sequence: Synthetic primer"
<400> 197
   tcctctctct gaaatcactg agct 24
<210> 198
   <211> 24
   <212> DNA
   <213> Artificial Sequence
<220>
   <221> source
   <223> /note="Description of Artificial Sequence: Synthetic primer"
<400> 198
   tcctctctct gaaatcactg agat 24
<210> 199
   <211> 24
   <212> DNA
   <213> Artificial Sequence
<220>
   <221> source
   <223> /note="Description of Artificial Sequence: Synthetic primer"
<400> 199
   tcctctctct gaaatcactg aggt 24
<210> 200
   <211> 24
   <212> DNA
   <213> Artificial Sequence
<220>
   <221> source
   <223> /note="Description of Artificial Sequence: Synthetic primer"
<400> 200
   tcctctctct gaaatcactg agtt 24
<210> 201
   <211> 24
   <212> DNA
   <213> Artificial Sequence
<220>
   <221> source
   <223> /note="Description of Artificial Sequence: Synthetic primer"
<400> 201
   tcctctctct gaaatcactg aact 24
<210> 202
   <211> 24
   <212> DNA
   <213> Artificial Sequence
<220>
   <221> source
   <223> /note="Description of Artificial Sequence: Synthetic primer"
<400> 202
   tcctctctct gaaatcactg acct 24
<210> 203
   <211> 24
   <212> DNA
   <213> Artificial Sequence
<220>
   <221> source
   <223> /note="Description of Artificial Sequence: Synthetic primer"
<400> 203
   tcctctctct gaaatcactg atct 24
<210> 204
   <211> 24
   <212> DNA
   <213> Artificial Sequence
<220>
   <221> source
   <223> /note="Description of Artificial Sequence: Synthetic primer"
<400> 204
   tcctctctct gaaatcactg cgct 24
<210> 205
   <211> 24
   <212> DNA
   <213> Artificial Sequence
<220>
   <221> source
   <223> /note="Description of Artificial Sequence: Synthetic primer"
<400> 205
   tcctctctct gaaatcactg ggct 24
<210> 206
   <211> 24
   <212> DNA
   <213> Artificial Sequence
<220>
   <221> source
   <223> /note="Description of Artificial Sequence: Synthetic primer"
<400> 206
   tcctctctct gaaatcactg tgct 24
<210> 207
   <211> 29
   <212> DNA
   <213> Artificial Sequence
<220>
   <221> source
   <223> /note="Description of Artificial Sequence: Synthetic primer"
<400> 207
   catgaaacaa atgaatgatg cacatcatg 29
<210> 208
   <211> 29
   <212> DNA
   <213> Artificial Sequence
<220>
   <221> source
   <223> /note="Description of Artificial Sequence: Synthetic primer"
<400> 208
   catgaaacaa atgaatgatg cacatcatc 29
<210> 209
   <211> 29
   <212> DNA
   <213> Artificial Sequence
<220>
   <221> source
   <223> /note="Description of Artificial Sequence: Synthetic primer"
<400> 209
   catgaaacaa atgaatgatg cacatcaac 29
<210> 210
   <211> 29
   <212> DNA
   <213> Artificial Sequence
<220>
   <221> source
   <223> /note="Description of Artificial Sequence: Synthetic primer"
<400> 210
   catgaaacaa atgaatgatg cacatcacc 29
<210> 211
   <211> 29
   <212> DNA
   <213> Artificial Sequence
<220>
   <221> source
   <223> /note="Description of Artificial Sequence: Synthetic primer"
<400> 211
   catgaaacaa atgaatgatg cacatcagc 29
<210> 212
   <211> 29
   <212> DNA
   <213> Artificial Sequence
<220>
   <221> source
   <223> /note="Description of Artificial Sequence: Synthetic primer"
<400> 212
   catgaaacaa atgaatgatg cacatcctc 29
<210> 213
   <211> 29
   <212> DNA
   <213> Artificial Sequence
<220>
   <221> source
   <223> /note="Description of Artificial Sequence: Synthetic primer"
<400> 213
   catgaaacaa atgaatgatg cacatcgtc 29
<210> 214
   <211> 29
   <212> DNA
   <213> Artificial Sequence
<220>
   <221> source
   <223> /note="Description of Artificial Sequence: Synthetic primer"
<400> 214
   catgaaacaa atgaatgatg cacatcttc 29
<210> 215
   <211> 29
   <212> DNA
   <213> Artificial Sequence
<220>
   <221> source
   <223> /note="Description of Artificial Sequence: Synthetic primer"
<400> 215
   catgaaacaa atgaatgatg cacataatc 29
<210> 216
   <211> 29
   <212> DNA
   <213> Artificial Sequence
<220>
   <221> source
   <223> /note="Description of Artificial Sequence: Synthetic primer"
<400> 216
   catgaaacaa atgaatgatg cacatgatc 29
<210> 217
   <211> 29
   <212> DNA
   <213> Artificial Sequence
<220>
   <221> source
   <223> /note="Description of Artificial Sequence: Synthetic primer"
<400> 217
   catgaaacaa atgaatgatg cacattatc 29
<210> 218
   <211> 23
   <212> DNA
   <213> Artificial Sequence
<220>
   <221> source
   <223> /note="Description of Artificial Sequence: Synthetic primer"
<400> 218
   agccaccatg atgtgcatca ttc 23
<210> 219
   <211> 23
   <212> DNA
   <213> Artificial Sequence
<220>
   <221> source
   <223> /note="Description of Artificial Sequence: Synthetic primer"
<400> 219
   agccaccatg atgtgcatca tta 23
<210> 220
   <211> 23
   <212> DNA
   <213> Artificial Sequence
<220>
   <221> source
   <223> /note="Description of Artificial Sequence: Synthetic primer"
<400> 220
   agccaccatg atgtgcatca taa 23
<210> 221
   <211> 23
   <212> DNA
   <213> Artificial Sequence
<220>
   <221> source
   <223> /note="Description of Artificial Sequence: Synthetic primer"
<400> 221
   agccaccatg atgtgcatca tga 23
<210> 222
   <211> 23
   <212> DNA
   <213> Artificial Sequence
<220>
   <221> source
   <223> /note="Description of Artificial Sequence: Synthetic primer"
<400> 222
   agccaccatg atgtgcatca ata 23
<210> 223
   <211> 23
   <212> DNA
   <213> Artificial Sequence
<220>
   <221> source
   <223> /note="Description of Artificial Sequence: Synthetic primer"
<400> 223
   agccaccatg atgtgcatca cta 23
<210> 224
   <211> 23
   <212> DNA
   <213> Artificial Sequence
<220>
   <221> source
   <223> /note="Description of Artificial Sequence: Synthetic primer"
<400> 224
   agccaccatg atgtgcatca gta 23
<210> 225
   <211> 23
   <212> DNA
   <213> Artificial Sequence
<220>
   <221> source
   <223> /note="Description of Artificial Sequence: Synthetic primer"
<400> 225
   agccaccatg atgtgcatcc tta 23
<210> 226
   <211> 23
   <212> DNA
   <213> Artificial Sequence
<220>
   <221> source
   <223> /note="Description of Artificial Sequence: Synthetic primer"
<400> 226
   agccaccatg atgtgcatcg tta 23
<210> 227
   <211> 23
   <212> DNA
   <213> Artificial Sequence
<220>
   <221> source
   <223> /note="Description of Artificial Sequence: Synthetic primer"
<400> 227
   agccaccatg atgtgcatct tta 23
<210> 228
   <211> 23
   <212> DNA
   <213> Artificial Sequence
<220>
   <221> source
   <223> /note="Description of Artificial Sequence: Synthetic primer"
<400> 228
   aggcaccatg atgtgcatca tta 23
<210> 229
   <211> 23
   <212> DNA
   <213> Artificial Sequence
<220>
   <221> source
   <223> /note="Description of Artificial Sequence: Synthetic primer"
<220>
   <221> modified_base
   <222> (23) .. (23)
   <223> N6-tert-butyl-benzyl-deoxyadenine
<400> 229
   aggcaccatg atgtgcatca tta 23
<210> 230
   <211> 23
   <212> DNA
   <213> Artificial Sequence
<220>
   <221> source
   <223> /note="Description of Artificial Sequence: Synthetic primer"
<220>
   <221> modified_base
   <222> (20) .. (20)
   <223> N6-tert-butyl-benzyl-deoxyadenine
<400> 230
   aggcaccatg atgtgcatca tta 23

## Description

### FIELD OF THE INVENTION

The invention relates to cancer diagnostics and companion diagnostics for cancer therapies. In particular, the invention relates to methods and compositions for detection of mutations that are useful for diagnosis and prognosis as well as predicting the effectiveness of treatment of cancer.

### BACKGROUND OF THE INVENTION

Phosphatidylinositol 3-kinases (PI3Ks) are intracellular lipid kinases that regulate signaling pathways controlling cell proliferation and survival, adhesion and motility (Vivanco and Sawyers, (2002) The phosphatidylinositol 3-Kinase AKT pathway in human cancer, Nature Rev. Cancer 2:489). PI3KCA (PIK3CA) is a member of the PI3K gene family encoding the catalytic subunit of the kinase p110α. This gene is of unique relevance for neoplasia: of all the PI3K genes tested, only PI3KCA was found mutated in multiple cancers. In one study, somatic mutations in the PI3KCA gene were found in 32% of colon cancers, 27% glioblastomas, 25% gastric cancers, 8% breast cancers and 4% lung cancers (Samuels et al. (2004) High frequency of mutations in the PI3KCA gene in human cancers, Science 304:554). Later studies reported mutations also in uterine (24%), ovarian (10%) and cervical (10%) cancer (Brana and Sui, (2012) Clinical development of phosphatidylinositol 3-kinase inhibitors for cancer treatment, BMC Medicine 2012, 10:161).

PI3K activates the intracellular Akt/mTOR pathway by specifically activating the Akt protein. A genetic approach revealed that constitutive activation of this pathway by the mutant PI3KCA contributes to resistance to EGFR targeting therapies (Berns et al. (2007) A functional genetic approach identifies the PI3K pathway as a major determinant of trastuzumab resistance in breast cancer, Cancer Cell 12:395). At the same time, it was demonstrated that an intact (non-mutated) PI3KCA activity may be suppressed by specific inhibitors thus overcoming the effect of the disregulated upstream element in the pathway (e.g. EGFR) and recently, therapeutic agents targeting PI3KCA (p110α) itself have been developed (reviewed in Weickhardt et al. (2010) Strategies for Overcoming Inherent and Acquired Resistance to EGFR Inhibitors by Targeting Downstream Effectors in the RAS/PI3K Pathway, Current Cancer Drug Targets, 10:824; and Brana and Sui, (2012) Clinical development of phosphatidylinositol 3-kinase inhibitors for cancer treatment, BMC Medicine 2012, 10:161).

Taken together, these studies demonstrate the need for methods and tools for detecting somatic mutations in the PI3KCA gene for delivering personalized healthcare to patients seeking targeted cancer therapies.

To date, over 30 somatic mutations in the PI3KCA gene have been identified (U.S. Patent No. 8,026,053; M. Gymnopoulos et al. (2007), Rare cancer-specific mutations in PIK3CA show gain of function, Proceedings of the National Academy of Sciences, 104:5569-5574). The majority of the mutations cluster in exons 9 and 20. However a number of clinically significant mutations have been reported in exons 1, 4 and 7 as well. A diagnostic assay should target as many of these mutations as possible. Furthermore, precise discrimination (high specificity) is required since the output of the assay will determine the course of a patient's cancer therapy.

Allele-specific primers for the detection of various PI3KCA mutants are described in the art, including H1047L and H1047R (see, e.g., WO2009/040557, WO2011/131151 and WO2011/087928). WO 2009/040557 further discloses to use additional primer mismatches close to the 3'-terminus to enhance the specificity of the reactions. US2010/0286143 describes reactions wherein a single base like labelled ddNTPs are added to allele-specific probes during the extension step; specific primers for allele-specific PCR are, however, not disclosed.

### SUMMARY OF THE INVENTION

The invention comprises oligonucleotides for detecting a mutation H1047Y in the human PIK3CA gene, that are at least 90% identical to and have the 3'-terminal nucleotide of SEQ ID NO: 39, which comprises 3 or fewer mismatches, excluding the 3'-terminal nucleotide, wherein at least one mismatch or at least one modified nucleotide is among the penultimate 5 nucleotides at the 3'-terminus.

In another embodiment, the invention is a method of assaying a sample for the presence of mutation H1047Y in the human PIK3CA gene comprising contacting the sample with one allele-specific nucleotide oligonucleotide sharing at least 90% identity with and having the same 3-terminal nucleotide as an oligonucleotide comprising SEQ ID NO: 39, which comprises 3 or fewer mismatches, excluding the 3'-terminal nucleotide, wherein at least one mismatch or at least one modified nucleotide is among the penultimate 5 nucleotides at the 3'-terminus. In variations of this embodiment, the oligonucleotide is selected from a group consisting of SEQ ID NOs: 8, 21, 46, 78, 93, 113, 141, 166, 170, 194, 199, 217 and 228.

In yet another embodiment, the invention is a set of oligonucleotides for detecting H1047Y and one or more mutations H1047L, H1047R, N345K, E542K, E545A, E545G, E545K, G1049R, M1043I, Q546E, Q546L and Q546K mutations in the PIK3CA gene comprising a combination of two or more oligonucleotides, wherein one oligonucleotide being at least 90% identical to and having the 3'-terminal nucleotide of SEQ ID NO: 39, which comprises 3 or fewer mismatches, excluding the 3'-terminal nucleotide, wherein at least one mismatch or at least one modified nucleotide is among the penultimate 5 nucleotides at the 3'-terminus and at least one allele-specific oligonucleotide for each mutation other than H0147Y is sharing at least 90% identity with and having the same 3-terminal nucleotide as an oligonucleotide selected from a group consisting of SEQ ID NOs: 2, 18, 61, 84, 100, 127, 148, 170, 185, 197, 208 and 219. In variations of this embodiment, the oligonucleotides are selected from SEQ ID NOs: 8, 21, 46, 78, 93, 113, 141, 166, 170, 194, 199, 217 and 228.

In yet another embodiment, the invention is a reaction mixture for detecting H1047Y and one or more mutations H1047L, H1047R, N345K, E542K, E545A, E545G, E545K, G1049R, M1043I, Q546E, Q546L and Q546K in the PIK3CA gene comprising two or more allele-specific oligonucleotides, wherein one oligonucleotide being at least 90% identical to and having the 3'-terminal nucleotide of SEQ ID NO: 39, which comprises 3 or fewer mismatches, excluding the 3'-terminal nucleotide, wherein at least one mismatch or at least one modified nucleotide is among the penultimate 5 nucleotides at the 3'-terminus and at least one allele-specific oligonucleotide for each mutation other than H0147Y is sharing at least 90% identity with and having the same 3-terminal nucleotide as an oligonucleotide selected from a group consisting of SEQ ID NOs: 2, 18, 61, 84, 100, 127, 148, 170, 185, 197, 208, 219. In variations of this embodiment, the reaction mixture comprises one or more of: SEQ ID NOs: 8, 21, 46, 78, 93, 113, 141, 166, 170, 194, 199, 217 and 228.

In yet another embodiment, the invention is a method of assessing cancer in a patient by detecting in the patient's sample mutation H1047Y and one or more mutations H1047L, H1047R, N345K, E542K, E545A, E545G, E545K, G1049R, M1043I, Q546E, Q546L and Q546K in the PIK3CA gene using two or more allele-specific oligonucleotides, wherein one oligonucleotide being at least 90% identical to and having the 3'-terminal nucleotide of SEQ ID NO: 39, which comprises 3 or fewer mismatches, excluding the 3'-terminal nucleotide, wherein at least one mismatch or at least one modified nucleotide is among the penultimate 5 nucleotides at the 3'-terminus and at least one allele-specific oligonucleotide for each mutation other than H0147Y is sharing at least 90% identity with and having the same 3-terminal nucleotide as an oligonucleotide selected from a group consisting of SEQ ID NOs: 2, 18, 61, 84, 100, 127, 148, 170, 185, 197, 208, 219. In variations of this embodiment, the one or more oligonucleotide is selected from: SEQ ID NOs: 8, 21, 46, 78, 93, 113, 141, 166, 170, 194, 199, 217 and 228.

### DETAILED DESCRIPTION OF THE INVENTION

### Definitions

To facilitate the understanding of this disclosure, the following definitions of the terms used herein are provided.

The term "X[n]Y" refers to a missense mutation that results in a substitution of amino acid X for amino acid Y at position [n] within the amino acid sequence. For example, the term "H1047R" refers to a mutation where histidine at position 1047 is replaced with arginine.

The term "allele-specific primer" or "AS primer" refers to a primer that hybridizes to more than one variant of the target sequence, but is capable of discriminating between the variants of the target sequence in that only with one of the variants, the primer is efficiently extended by the nucleic acid polymerase under suitable conditions. With other variants of the target sequence, the extension is less efficient or inefficient.

The term "common primer" refers to the second primer in the pair of primers that includes an allele-specific primer. The common primer is not allele-specific, i.e. does not discriminate between the variants of the target sequence between which the allele-specific primer discriminates.

The term "assessing" in connection with cancer refers to inferring the status or condition of the cancer as well as determining the need for diagnostic procedures or treatments, evaluating potential effectiveness of the treatments, monitoring the subject's cancer, or any other steps or processes related to treatment or diagnosis of a cancer.

The terms "complementary" or "complementarity" are used in reference to antiparallel strands of polynucleotides related by the Watson-Crick base-pairing rules. The terms "perfectly complementary" or "100% complementary" refer to complementary sequences that have Watson-Crick pairing of all the bases between the antiparallel strands, i.e. there are no mismatches between any two bases in the polynucleotide duplex. However, duplexes are formed between antiparallel strands even in the absence of perfect complementarity. The terms "partially complementary" or "incompletely complementary" refer to any alignment of bases between antiparallel polynucleotide strands that is less than 100% perfect (e.g., there exists at least one mismatch or unmatched base in the polynucleotide duplex). The duplexes between partially complementary strands are generally less stable than the duplexes between perfectly complementary strands.

The term "sample" refers to any composition containing or presumed to contain nucleic acid. This includes a sample of tissue or fluid isolated from an individual for example, skin, plasma, serum, spinal fluid, lymph fluid, synovial fluid, urine, tears, blood cells, organs and tumors, and also to samples of *in vitro* cultures established from cells taken from an individual, including the formalin-fixed paraffin embedded tissues (FFPET) and nucleic acids isolated therefrom. To detect a somatic mutation, the sample is typically comprises a fragment of a solid tumor (primary or metastatic) or tumor-derived cells found elsewhere in the body, e.g. in circulating blood.

The terms "polynucleotide" and "oligonucleotide" are used interchangeably. "Oligonucleotide" is a term sometimes used to describe a shorter polynucleotide. An oligonucleotide may be comprised of at least 6 nucleotides, for example at least about 10-12 nucleotides, or at least about 15-30 nucleotides corresponding to a region of the designated nucleotide sequence.

The term "primary sequence" refers to the sequence of nucleotides in a polynucleotide or oligonucleotide. Nucleotide modifications such as nitrogenous base modifications, sugar modifications or other backbone modifications are not a part of the primary sequence. Labels, such as chromophores conjugated to the oligonucleotides are also not a part of the primary sequence. Thus two oligonucleotides can share the same primary sequence but differ with respect to the modifications and labels.

The term "primer" refers to an oligonucleotide which hybridizes with a sequence in the target nucleic acid and is capable of acting as a point of initiation of synthesis along a complementary strand of nucleic acid under conditions suitable for such synthesis. As used herein, the term "probe" refers to an oligonucleotide which hybridizes with a sequence in the target nucleic acid and is usually detectably labeled. The probe can have modifications, such as a 3'-terminus modification that makes the probe non-extendable by nucleic acid polymerases, and one or more chromophores. An oligonucleotide with the same sequence may serve as a primer in one assay and a probe in a different assay.

The term "modified nucleotide" refers to a unit in a nucleic acid polymer that contains a modified base, sugar or phosphate group, or that incorporates a non-natural moiety in its structure. Examples of non-natural nucleotides, include nucleotides with a modified nitrogenous base, e.g. alkylated or otherwise substitutes with a group not present among the conventional nitrogenous bases involved in Watson-Crick pairing. By way of illustration and not limitation, modified nucleotides include those with bases substituted with methyl, ethyl, benzyl or butyl-benzyl groups.

As used herein, the term "target sequence", "target nucleic acid" or "target" refers to a portion of the nucleic acid sequence which is to be either amplified, detected or both.

The terms "hybridized" and "hybridization" refer to the base-pairing interactions between two nucleic acids that result in formation of a duplex. It is not a requirement that two nucleic acids have 100% complementarity over their full length to achieve hybridization.

The present invention comprises methods and compositions for rapid and precise determination of the presence of mutation H1047Y and one or more of other mutations in the PI3KCA gene in patient's samples. The invention enables detection of mutation H1047Y and optionally one or more of the mutations selected from H1047L, H1047R, N345K, E542K, E545A, E545G, E545K, G1049R, M1043I, Q546E, Q546L and Q546K as well as a simultaneous query for 1, 2, 3, 4, 5, 6, 7, 8, 9, 10, 11, 12 or 13 of the mutations listed above.

One technique that is sensitive and amenable to multiplexing is allele-specific PCR (AS-PCR) described in e.g. U.S. Patent No. 6,627,402. This technique detects mutations or polymorphisms in nucleic acid sequences in the presence of wild-type variants of the sequences. In a successful allele-specific PCR, the desired variant of the target nucleic acid is amplified, while the other variants are not, at least not to a detectable level.

One measure of discrimination of an allele-specific PCR is the difference between Cₜ values (ΔCₜ) in the amplification reactions involving the two alleles. Each amplification reaction is characterized by a "growth curve" or "amplification curve" in the context of a nucleic acid amplification assay is a graph of a function, where an independent variable is the number of amplification cycles and a dependent variable is an amplification-dependent measurable parameter measured at each cycle of amplification, such as fluorescence emitted by a fluorophore. Typically, the amplification-dependent measurable parameter is the amount of fluorescence emitted by the probe upon hybridization, or upon the hydrolysis of the probe by the nuclease activity of the nucleic acid polymerase, see Holland et al., (1991) Proc. Natl. Acad. Sci. 88:7276-7280 and U.S. Patent No. 5,210,015. A growth curve is characterized by a "threshold value" (or Ct value) which is a number of cycles where a predetermined magnitude of the measurable parameter is achieved. A lower Ct value represents more rapid amplification, while the higher Ct value represents slower amplification. In the context of an allele-specific reaction the difference between Ct values of the two templates represents allelic discrimination in the reaction.

In an allele-specific PCR, at least one primer is allele-specific such that primer extension occurs only (or preferentially) when the specific variant of the sequence is present and does not occur (or occurs less efficiently, i.e. with a substantial ΔCₜ) when another variant is present. Design of successful allele-specific primers is an unpredictable art. While it is routine to design a primer for a known sequence, no formula exists for designing a primer that can discriminate between very similar sequences. The discrimination is especially challenging when one or more allele-specific primers targeting one or more polymorphic sites are present in the same reaction mixture.

Typically, the discriminating nucleotide in the primer, i.e. the nucleotide matching only one variant of the target sequence, is the 3'-terminal nucleotide. However, the 3' terminus of the primer is only one of many determinants of specificity. For example, additional mismatches may also affect discrimination (see U.S. Patent Application Publ. No. US20100099110). Another approach is to include non-natural or modified nucleotides that alter base pairing between the primer and the target sequence (U.S. Patent No. 6,001,611). The reduced extension kinetics and thus specificity of a primer is influenced by many factors including overall sequence context of the mismatch and other nucleic acids present in the reaction. The effect of these external factors on each additional mismatch as well as of each additional non-natural nucleotide either alone or in combination cannot be predicted. The applicants tested multiple variants of the primers and found that surprisingly, certain variants are dramatically different with respect to their ability to discriminate between closely related target sequences.

For successful extension of a primer, complementarity at the 3'-end of the primer is more critical than complementarity at the 5'-end of the primer (Innis et al. Eds. PCR Protocols, (1990) Academic Press, Chapter 1, pp. 9-11). Therefore the present invention encompasses the primers disclosed in Tables 1-13 as well as equivalents thereof with 5'-end variations.

In one embodiment the present invention comprises oligonucleotides for detecting PI3KCA mutation H1047Y and optionally PI3KCA mutations selected from H1047L, H1047R, N345K, E542K, E545A, E545G, E545K, G1049R, M1043I, Q546E, Q546L and Q546K as well as a simultaneous query for 1, 2, 3, 4, 5, 6, 7, 8, 9, 10, 11, 12 or 13 of the mutations listed above. In one embodiment, the invention comprises an oligonucleotide being at least 90% identical to and having the 3'-terminal of SEQ ID NO: 39, which comprises 3 or fewer mismatches, excluding the 3'-terminal nucleotide, wherein at least one mismatch or at least one modified nucleotide is among the penultimate 5 nucleotides at the 3'-terminus and optionally one or more oligonucleotides selected from SEQ ID NOs: 2, 18, 61, 84, 100, 127, 148, 170, 185, 197, 208 and 219 (Tables 1-13) as well as variations at least 90% identical to and having the 3'-terminal nucleotide of said oligonucleotides, for specifically detecting mutations in the human PI3KCA gene. As illustrated in Tables 1-13, oligonucleotides sharing 90% identity with a given oligonucleotide include those having 1, 2 or 3 mismatches with that oligonucleotide. As further illustrated in Tables 1-13, oligonucleotides sharing 90% identity with a given oligonucleotide also include those having one or more non-natural nucleotide. As further illustrated in Tables 1-13, the mismatches and non-natural nucleotides typically occur within the 3'-terminal portion of the oligonucleotide, specifically within 5 penultimate nucleotides. However, some oligonucleotides sharing 90% identity with a given oligonucleotide also include those having 1, 2 or 3 mismatches elsewhere in the oligonucleotide, e.g. in the 5'-portion of the oligonucleotide. As demonstrated in examples below, the oligonucleotides of the present invention are characterized by a substantial positive ΔCₜ determined using the formula ΔCₜ = Cₜ(wild type) - Cₜ(mutant), indicating that amplification of the wild-type template is detectably slower than that of the mutant template.

### Legends to the tables

The underlined nucleotides are mismatched with both the wild-type and the mutant sequence. The following abbreviations are used for the modified-base nucleotides: A* and C* are respectively N6-tert-butyl-benzyl-deoxyadenine and N4-tert-butyl-benzyl-deoxycytosine, C^ is N4-ethyl- deoxycytosine; and C^{#} is N4-methyl-deoxycytosine.

**Table 1: Oligonucleotides for detecting mutation H1047L**

| SEQ ID NO: | SEQUENCE 5'-3' |
|---|---|
| 1 | TTTTGTTGTCCAGCCACCATGAT |
| 2 | TTTTGTTGTCCAGCCACCATGAA |
| 3 | TTTTGTTGTCCAGCCACCATGCA |
| 4 | TTTTGTTGTCCAGCCACCATGGA |
| 5 | TTTTGTTGTCCAGCCACCATGTA |
| 6 | TTTTGTTGTCCAGCCACCATCAA |
| 7 | TTTTGTTGTCCAGCCACCATTAA |
| 8 | TTTTGTTGTCCAGCCACCATAAA |
| 9 | TTTTGTTGTCCAGCCACCAAGAA |
| 10 | TTTTGTTGTCCAGCCACCACGAA |
| 11 | TTTTGTTGTCCAGCCACCAGGAA |
| 12 | GTTTTTGTTGTCCAGCCACCATGAA |
| 13 | GTTTTTGTTGTCCAGCCACCATGAA*** |
| 14 | GTTTTTGTTGTCCAGCCACCATGA***A |
| 15 | CC^{#}GTTTTTGTTGTC^{#}CAGC^{#}CACC^{#}ATGA***A |
| 16 | AATCC^{#}ATTGTTGTTGTC^{#}CAGC^{#}CACC^{#}ATGAA*** |

**Table 2: Oligonucleotides for detecting mutation H1047R**

| SEQ ID NO: | SEQUENCE 5'-3' |
|---|---|
| 17 | TTTGTTGTCCAGCCACCATGAT |
| 18 | TTTGTTGTCCAGCCACCATGCC |
| 19 | TTTGTTGTCCAGCCACCATGGC |
| 20 | TTTGTTGTCCAGCCACCATGTC |
| 21 | TTTGTTGTCCAGCCACCATCAC |
| 22 | TTTGTTGTCCAGCCACCATTAC |
| 23 | TTTGTTGTCCAGCCACCATAAC |
| 24 | TTTGTTGTCCAGCCACCAAGAC |
| 25 | TTTGTTGTCCAGCCACCACGAC |
| 26 | TTTGTTGTCCAGCCACCAGGAC |
| 27 | TTTGTTGTCCAGCCACCATGAT |
| 28 | TTTGTTGTCCAGCCACCATGCC |
| 29 | TTTCATGAAACAAATGAATGATGCAGG |
| 30 | TTTCATGAAACAAATGAATGATGCATG |
| 31 | TTTCATGAAACAAATGAATGATGCAAG |
| 32 | TTTCATGAAACAAATGAATGATGCCCG |
| 33 | TTTCATGAAACAAATGAATGATGCGCG |
| 34 | TTTCATGAAACAAATGAATGATGCTCG |
| 35 | TTTCATGAAACAAATGAATGATGGACG |
| 36 | TTTCATGAAACAAATGAATGATGTACG |
| 37 | TTTCATGAAACAAATGAATGATGAACG |

**Table 3: Oligonucleotides for detecting mutation H1047Y**

| SEQ ID NO: | SEQUENCE 5'-3' |
|---|---|
| 38 | TTTGTTGTCCAGCCACCATGATG |
| 39 | TTTGTTGTCCAGCCACCATGAAA |
| 40 | TTTGTTGTCCAGCCACCATGACA |
| 41 | TTTGTTGTCCAGCCACCATGAGA |
| 42 | TTTGTTGTCCAGCCACCATGCTA |
| 43 | TTTGTTGTCCAGCCACCATGGTA |
| 44 | TTTGTTGTCCAGCCACCATGTTA |
| 45 | TTTGTTGTCCAGCCACCATCATA |
| 46 | TTTGTTGTCCAGCCACCATTATA |
| 47 | TTTGTTGTCCAGCCACCATAATA |
| 48 | GTTTTGTTGTCCAGCCACCATGA***TA |
| 49 | TTGTGTTGTCCAGCCACCATGA***TA |
| 50 | AGTATTTCATGAAACAAATGAATGATGCGT |
| 51 | AGTATTTCATGAAACAAATGAATGATGCTT |
| 52 | AGTATTTCATGAAACAAATGAATGATGGAT |
| 53 | AGTATTTCATGAAACAAATGAATGATGTAT |
| 54 | AGTATTTCATGAAACAAATGAATGATGAAT |
| 55 | AGTGTTTCATGAAACAAATGAATGATGCA***T |
| 56 | AGTGTTTCATGAAACAAATGAATGATGC***AT |
| 57 | AGTATTTCATGAAACAAATGAATGATGC^GT |
| 58 | AGTATTTCATGAAACAAATGAATGATTCA***T |
| 59 | AGTATTTCATGAAACAAATGAATGATGC^TT |

**Table 4: Oligonucleotides for detecting mutation N345K**

| SEQ ID NO: | SEQUENCE 5'-3' |
|---|---|
| 60 | ATAAAAATTCTTTGTGCAACCTACGTGAAT |
| 61 | ATAAAAATTCTTTGTGCAACCTACGTGAAA |
| 62 | ATAAAAATTCTTTGTGCAACCTACGTGACA |
| 63 | ATAAAAATTCTTTGTGCAACCTACGTGAGA |
| 64 | ATAAAAATTCTTTGTGCAACCTACGTGATA |
| 65 | ATAAAAATTCTTTGTGCAACCTACGTGCAA |
| 66 | ATAAAAATTCTTTGTGCAACCTACGTGGAA |
| 67 | ATAAAAATTCTTTGTGCAACCTACGTGTAA |
| 68 | ATAAAAATTCTTTGTGCAACCTACGTCAAA |
| 69 | ATAAAAATTCTTTGTGCAACCTACGTTAAA |
| 70 | ATAAAAATTCTTTGTGCAACCTACGTAAAA |
| 71 | ATAGAAATTCTTTGTGCAACCTACGTGAAA |
| 72 | ATGAAAATTCTTTGTGCAACCTACGTGAAA* |
| 73 | ATGAAAATTCTTTGTGCAACCTACGTGAA*A |
| 74 | ATGAAAATTCTTTGTGCAACCTACGTGA*AA |
| 75 | ATAAAAATTCTTTGTGCAACCTACGTGAC*A |
| 76 | ATAAAAATTCTTTGTGCAACCTACGTGC*AA |
| 77 | ATAAAAATTCTTTGTGCAACCTACGTGAC#A |
| 78 | ATAAAAATTCTTTGTGCAACCTACGGGAAA* |
| 79 | ATAAAAATTCTTTGTGCAACCTACGTC*AAA |
| 80 | ATAAAAATTCTTTGTGCAACCTACGGGAA*A |
| 81 | ATAAAAATTCTTTGTGCAACCTACGTC#AAA |
| 82 | ATAAAAATTCTTTGTGCAACCTACGTC#AAA* |

**Table 5: Oligonucleotides for detecting mutation E542K**

| SEQ ID NO: | SEQUENCE 5'-3' |
|---|---|
| 83 | CAATTTCTACACGAGATCCTCTCTCTG |
| 84 | CAATTTCTACACGAGATCCTCTCTCTA |
| 85 | CAATTTCTACACGAGATCCTCTCTCAA |
| 86 | CAATTTCTACACGAGATCCTCTCTCCA |
| 87 | CAATTTCTACACGAGATCCTCTCTCGA |
| 88 | CAATTTCTACACGAGATCCTCTCTGTA |
| 89 | CAATTTCTACACGAGATCCTCTCTTTA |
| 90 | CAATTTCTACACGAGATCCTCTCTATA |
| 91 | CAATTTCTACACGAGATCCTCTCACTA |
| 92 | CAATTTCTACACGAGATCCTCTCCCTA |
| 93 | CAATTTCTACACGAGATCCTCTCGCTA |
| 94 | CAGTTTCTACACGAGATCCTCTCTCTA |
| 95 | GAAGCAATTTCTACACGAGATCCTCTCTCTA* |
| 96 | GAAGCAATTTCTACACGAGATCCTCTCTC*TA |
| 97 | CAGTTTCTACACGAGATCCTCTCTC*TA |

**Table 6: Oligonucleotides for detecting mutation E545A**

| SEQ ID NO: | SEQUENCE 5'-3' |
|---|---|
| 98 | GAGATCCTCTCTCTGAAATCACTGA |
| 99 | GAGATCCTCTCTCTGAAATCACTGC |
| 100 | GAGATCCTCTCTCTGAAATCACTCC |
| 101 | GAGATCCTCTCTCTGAAATCACTTC |
| 102 | GAGATCCTCTCTCTGAAATCACTAC |
| 103 | GAGATCCTCTCTCTGAAATCACAGC |
| 104 | GAGATCCTCTCTCTGAAATCACCGC |
| 105 | GAGATCCTCTCTCTGAAATCACGGC |
| 106 | GAGATCCTCTCTCTGAAATCAGTGC |
| 107 | GAGATCCTCTCTCTGAAATCATTGC |
| 108 | GAGATCCTCTCTCTGAAATCAATGC |
| 109 | GGGATCCTCTCTCTGAAATCACTGC |
| 110 | GGGATCCTCTCTCTGAAATCAC*TGC |
| 111 | GGGATCCTCTCTCTGAAATCACTGC* |
| 112 | GAGATCCTCTCTCTGAAATCGCTGC* |
| 113 | GAGATCCTCTCTCTGAAATCATTGC* |
| 114 | GAGATCCTCTCTCTGAAATCACTC*C |
| 115 | GAGATCCTCTCTCTGAAATCA*CTLC |
| 116 | GAGATCCTCTCTCTGAAATCACC*GC |
| 117 | GAGATCCTCTCTCTGAAATCACTA*C |
| 118 | GAGATCCTCTCTCTGAAATCGCC^GC |
| 119 | GAGATCCTCTCTCTGAAATCACCGC* |
| 120 | GAGATCCTCTCTCTGAAATCACGGC* |
| 121 | GAGATCCTCTCTCTGAAATCAC^TC^C |
| 122 | GAGATCCTCTCTCTGAAATCAC^GGC |
| 123 | GAGATCCTCTCTCTGAAATCAC*CGC |
| 124 | GAGATCCTCTCTCTGAAATCACA*GC |

| | |
|---|---|
| L-Gclamp | |

**Table 7: Oligonucleotides for detecting mutation E545G**

| SEQ ID NO: | SEQUENCE 5'-3' |
|---|---|
| 125 | GAGATCCTCTCTCTGAAATCACTGA |
| 126 | GAGATCCTCTCTCTGAAATCACTGG |
| 127 | GAGATCCTCTCTCTGAAATCACTCG |
| 128 | GAGATCCTCTCTCTGAAATCACTAG |
| 129 | GAGATCCTCTCTCTGAAATCACTTG |
| 130 | GAGATCCTCTCTCTGAAATCACAGG |
| 131 | GAGATCCTCTCTCTGAAATCACCGG |
| 132 | GAGATCCTCTCTCTGAAATCACGGG |
| 133 | GAGATCCTCTCTCTGAAATCAGTGG |
| 134 | GAGATCCTCTCTCTGAAATCAATGG |
| 135 | GAGATCCTCTCTCTGAAATCATTGG |
| 136 | GGGATCCTCTCTCTGAAATCACTGG |
| 137 | GGGATCCTCTCTCTGAAATCAC*TGG |
| 138 | GAGATCCTCTCTCTGAAATCACTC*G |
| 139 | GAGATCCTCTCTCTGAAATCA*CTC^G |
| 140 | GAGATCCTCTCTCTGAAATCA*CTTG |
| 141 | GAGATCCTCTCTCTGAAATCACTA*G |
| 142 | GAGATCCTCTCTCTGAAA TCACTC^G |
| 143 | GAGATCCTCTCTCTGAAATCAA*TGG |
| 144 | CTATACGAGATCCTCTCTCTIAAATCAC*TGG |
| 145 | AGATCCTCTCTCTGAAATCACTAG |
| 146 | AGA TCCTCTCTCTGAAATCACGGG |

**Table 8: Oligonucleotides for detecting mutation E545K**

| SEQ ID NO: | SEQUENCE 5'-3' |
|---|---|
| 147 | ACGAGATCCTCTCTCTGAAATCACTG |
| 148 | ACGAGATCCTCTCTCTGAAATCACTA |
| 149 | ACGAGATCCTCTCTCTGAAATCACAA |
| 150 | ACGAGATCCTCTCTCTGAAATCACCA |
| 151 | ACGAGATCCTCTCTCTGAAATCACGA |
| 152 | ACGAGA TCCTCTCTCTGAAA TCAGTA |
| 153 | ACGAGATCCTCTCTCTGAAATCAATA |
| 154 | ACGAGATCCTCTCTCTGAAATCATTA |
| 155 | ACGAGATCCTCTCTCTGAAATCCCTA |
| 156 | ACGAGATCCTCTCTCTGAAATCGCTA |
| 157 | ACGAGATCCTCTCTCTGAAATCTCTA |
| 158 | AGGAGATCCTCTCTCTGAAATCACTA |
| 159 | AGGAGATCCTCTCTCTGAAATCACTA* |
| 160 | AGGAGATCCTCTCTCTGAAATCAC^TA |
| 161 | AGGAGATCCTCTCTCTGAAATCA*CTA |
| 162 | ACGAGATCCTCTCTCTGAAATCAA*TA |
| 163 | ACGAGATCCTCTCTCTGAAATCACA*A |
| 164 | ACGAGATCCTCTCTCTGAAATC#AA*TA |
| 165 | ACGAGATCCTCTCTCTGAAATCACC*A |
| 166 | ACGAGATCCTCTCTCTGAAATCC*CTA |

**Table 9: Oligonucleotides for detecting mutation Q546K**

| SEQ ID NO: | SEQUENCE 5'-3' |
|---|---|
| 167 | AGATCCTCTCTCTGAAATCACTGAGC |
| 168 | AGATCCTCTCTCTGAAATCACTGAGA |
| 169 | AGATCCTCTCTCTGAAATCACTGACA |
| 170 | AGATCCTCTCTCTGAAATCACTGAAA |
| 171 | AGATCCTCTCTCTGAAATCACTGATA |
| 172 | AGATCCTCTCTCTGAAATCACTGCGA |
| 173 | AGATCCTCTCTCTGAAATCACTGTGA |
| 174 | AGATCCTCTCTCTGAAATCACTGGGA |
| 175 | AGATCCTCTCTCTGAAATCACTCAGA |
| 176 | AGATCCTCTCTCTGAAATCACTAAGA |
| 177 | AGATCCTCTCTCTGAAATCACTTAGA |
| 178 | AGGTCCTCTCTCTGAAATCACTGAGA |
| 179 | GAGGTCCTCTCTCTGAAATCACTGAGA* |
| 180 | GAGGTCCTCTCTCTGAAATCACTGA*GA |
| 181 | GAGATCCTCTCTCTGAAATCACTGAAA |
| 182 | GAGATCCTCTCTCTGAAATCACTGGGA |
| 183 | GAGATCCTCTCTCTGAAATCACTAAGA |

**Table 10: Oligonucleotides for detecting mutation Q546E**

| SEQ ID NO: | SEQUENCE 5'-3' |
|---|---|
| 184 | A TCCTCTCTCTGAAATCACTGAGC |
| 185 | ATCCTCTCTCTGAAATCACTGAGG |
| 186 | ATCCTCTCTCTGAAATCACTGAAG |
| 187 | ATCCTCTCTCTGAAATCACTGACG |
| 188 | ATCCTCTCTCTGAAATCACTGATG |
| 189 | ATCCTCTCTCTGAAATCACTGCGG |
| 190 | ATCCTCTCTCTGAAATCACTGGGG |
| 191 | ATCCTCTCTCTGAAATCACTGTGG |
| 192 | ATCCTCTCTCTGAAATCACTAAGG |
| 193 | ATCCTCTCTCTGAAATCACTCAGG |
| 194 | ATCCTCTCTCTGAAATCACTTAGG |

**Table 11: Oligonucleotides for detecting mutation Q546L**

| SEQ ID NO: | SEQUENCE 5'-3' |
|---|---|
| 195 | TCCTCTCTCTGAAATCACTGAGCA |
| 197 | TCCTCTCTCTGAAATCACTGAGCT |
| 198 | TCCTCTCTCTGAAATCACTGAGAT |
| 199 | TCCTCTCTCTGAAATCACTGAGGT |
| 200 | TCCTCTCTCTGAAATCACTGAGTT |
| 201 | TCCTCTCTCTGAAATCACTGAACT |
| 202 | TCCTCTCTCTGAAATCACTGACCT |
| 203 | TCCTCTCTCTGAAATCACTGATCT |
| 204 | TCCTCTCTCTGAAATCACTGCGCT |
| 205 | TCCTCTCTCTGAAATCACTGGGCT |
| 206 | TCCTCTCTCTGAAATCACTGTGCT |

**Table 12: Oligonucleotides for detecting mutation G1049R**

| SEQ ID NO: | SEQUENCE 5'-3' |
|---|---|
| 207 | CATGAAACAAATGAATGATGCACATCATG |
| 208 | CATGAAACAAATGAATGATGCACATCATC |
| 209 | CATGAAACAAATGAATGATGCACATCAAC |
| 210 | CATGAAACAAATGAATGATGCACATCACC |
| 211 | CATGAAACAAATGAATGATGCACATCAGC |
| 212 | CATGAAACAAATGAATGATGCACATCCTC |
| 213 | CATGAAACAAATGAATGATGCACATCGTC |
| 214 | CATGAAACAAATGAATGATGCACATCTTC |
| 215 | CATGAAACAAATGAATGATGCACATAATC |
| 216 | CATGAAACAAATGAATGATGCACATGATC |
| 217 | CATGAAACAAATGAATGATGCACATTATC |

**Table 13: Oligonucleotides for detecting mutation M1043I**

| SEQ ID NO: | SEQUENCE 5'-3' |
|---|---|
| 218 | AGCCACCATGATGTGCATCATTC |
| 219 | AGCCACCATGATGTGCATCATTA |
| 220 | AGCCACCATGATGTGCATCATAA |
| 221 | AGCCACCATGATGTGCATCATGA |
| 222 | AGCCACCATGATGTGCATCAATA |
| 223 | AGCCACCATGATGTGCATCACTA |
| 224 | AGCCACCATGATGTGCATCAGTA |
| 225 | AGCCACCATGATGTGCATCCTTA |
| 226 | AGCCACCATGATGTGCATCGTTA |
| 227 | AGCCACCATGATGTGCATCTTTA |
| 228 | AGGCACCATGATGTGCATCATTA |
| 229 | AGGCACCATGATGTGCATCATTA* |
| 230 | AGGCACCATGATGTGCATCA*TTA |

The present invention is further related to an oligonucleotide for detecting a mutation at one or more nucleotide position(s) between codons 1042 and 1050 in the PIK3CA gene being at least 90% identical to and having the 3'-terminal nucleotide of SEQ ID NO: 39 and of one or more of the sequences selected from the group consisting of SEQ ID NOs: 2, 18, 208 and 219. The oligonucleotides comprise 3 or fewer mismatches with one of said sequences, excluding the 3'-terminal nucleotide and/or at least one mismatch among the penultimate 5 nucleotides at the 3'-terminus. The oligonucleotides further comprise at least one modified nucleotide among the terminal 5 nucleotides at the 3'-terminus. The oligonucleotides are in particular suitable for detecting mutation H1047Y and one or more of the mutations M1043I, H1047L, H1047R, and/or H1049R. Further described is an oligonucleotide for detecting mutation N345K in the PIK3CA gene being at least 90% identical to and having the 3'-terminal nucleotide of SEQ ID NO: 61. The oligonucleotides might comprise 3 or fewer mismatches with SEQ ID NO: 61, excluding the 3'-terminal nucleotide and/or at least one mismatch among the penultimate 5 nucleotides at the 3'-terminus. The oligonucleotides might further comprise at least one modified nucleotide among the terminal 5 nucleotides at the 3'-terminus.

Another embodiment described is an oligonucleotide for detecting a mutation at one or more nucleotide position(s) between codons 541 and 547 in the PIK3CA gene being at least 90% identical to and having the 3'-terminal nucleotide of one or more of the sequences selected from the group consisting of SEQ ID NOs: 84, 99, 126, 148, 168, 185 and 197. The oligonucleotides might comprise 3 or fewer mismatches with one of said sequences, excluding the 3'-terminal nucleotide and/or at least one mismatch among the penultimate 5 nucleotides at the 3'-terminus. The oligonucleotides might further comprise at least one modified nucleotide among the terminal 5 nucleotides at the 3'-terminus. The oligonucleotides are in particular suitable for detecting one or more of the mutations E542K, E545A, E545G, E545K, Q546K, Q546L and/or Q546E.

In another embodiment, the present invention is a diagnostic method of detecting mutation H1047Y in the human PI3KCA (PIK3CA) gene and one or more mutations selected from H1047L, H1047R, N345K, E542K, E545A, E545G, E545K, G1049R, M1043I, Q546E, Q546L and Q546K as well as a simultaneous query for 1, 2, 3, 4, 5, 6, 7, 8, 9, 10, 11, 12 or 13 of the mutations listed above using oligonucleotide SEQ ID NO: 39 and optionally one or more of the oligonucleotides selected from SEQ ID NOs: 2, 18, 61, 84, 99, 126, 148, 168, 185, 197, 208, 219 or variations at least 90% identical to and having the 3'-terminal nucleotide of said oligonucleotides. In variations of this embodiment, the method comprises using one or more oligonucleotides selected from SEQ ID NOs: 8, 21, 46, 78, 93, 113, 141, 166, 170, 194, 199, 217 and 228. The method comprises contacting a test sample containing nucleic acids with one or more of the oligonucleotides in the presence of the corresponding downstream primer and a detection probe. Advantageously, detection of closely positioned mutations can be performed in a single reaction. In some embodiments, a single reaction contains two or more allele-specific oligonucleotides, e.g., SEQ ID NOs: 39 and 8, 21 and 46 can be combined in one reaction mixture together with a single downstream primer and a single detection probe. Similarly, a single reaction may contain two or more of SEQ ID NOs: 39 and 93, 113, 141, 166, 170 and 199 can be combined in one reaction mixture together with a single downstream primer and a single detection probe. The method comprises contacting a test sample containing nucleic acids with one or more of the oligonucleotides in the presence of the corresponding downstream primer (i.e. a primer capable of hybridizing to the opposite strand of the target nucleic acid so as to enable exponential amplification), nucleoside triphosphates and a nucleic acid polymerase, such that the one or more allele-specific primers is efficiently extended only when an PI3KCA mutation is present in the sample; and detecting the presence or absence of an PI3KCA mutation by directly or indirectly detecting the presence or absence of the primer extension.

In a particular embodiment the presence of the primer extension is detected with a probe. The probe may be labeled with a radioactive, or a chromophore (fluorophore) label, e.g. a label incorporating FAM, JA270, CY5 family dyes, or HEX dyes. As one example of detection using a fluorescently labeled probe, the mutation may be detected by real-time polymerase chain reaction (rt-PCR), where hybridization of the probe results in enzymatic digestion of the probe and detection of the resulting fluorescence (TaqMan™ probe method, Holland et al. (1991) P.N.A.S. USA 88:7276-7280). Alternatively, the presence of the extension product and the amplification product may be detected by gel electrophoresis followed by staining or by blotting and hybridization as described e.g., in Sambrook, J. and Russell, D.W. (2001) Molecular Cloning, 3rd ed. CSHL Press, Chapters 5 and 9.

In another embodiment, the invention is related to a method of treating a patient having a tumor possibly harboring cells with a mutant PI3KCA gene. The method comprises contacting a sample from the patient with two or more oligonucleotides, wherein one oligonucleotide is SEQ ID NO: 39 and at least one other oligonucleotide is selected from SEQ ID NOs: 2, 18, 61, 84, 100 (99), 127 (126), 148, 170 (168), 185, 197, 208, 219 or variations at least 90% identical to and having the 3'-terminal nucleotide of said oligonucleotides, in the presence of a corresponding second primer or primers, conducting allele-specific amplification, and detecting the presence or absence of an PI3KCA mutation by detecting presence or absence of the primer extension, and if at least one mutation is found or not found, subjecting the patient the appropriate treatment regimen. In some embodiments, the treatment comprises administering an inhibitor of the protein encoded by PI3KCA gene (p110-alpha protein). In other embodiments, the treatment comprises administering an inhibitor of a protein upstream in the pathway, e.g. the EGFR protein, if PI3KCA mutations are not found and administering an alternative treatment if the mutations are found. In variations of this embodiment, the method comprises contacting a sample from the patient with one or more oligonucleotides selected from SEQ ID NOs: 8, 21, 46, 78, 93, 113, 141, 166, 170, 194, 199, 217 and 228.

In yet another embodiment, the invention is a kit containing reagents for detecting mutations in the PI3KCA gene, specifically mutation H1047Y and one or more of the mutations selected from H1047L, H1047R, N345K, E542K, E545A, E545G, E545K, G1049R, M1043I, Q546E, Q546L and Q546K as well as a simultaneous query for 1, 2, 3, 4, 5, 6, 7, 8, 9, 10, 11, 12 or 13 of the mutations listed above. The reagents comprise two or more oligonucleotides, wherein one oligonucleotide is SEQ ID NO: 39 and at least one oligonucleotide is selected from SEQ ID NOs: 2, 18, 61, 84, 100 (99), 127 (126), 148, 170 (168), 185, 197, 208, 219 or variations at least 90% identical to and having the 3'-terminal nucleotide of said oligonucleotides, one or more corresponding second primers, and optionally, one or more probes. In variations of this embodiment, the reagents comprise SEQ ID NO: 39 and one or more oligonucleotides selected from SEQ ID NOs: 11, 32, 46, 78, 93, 113, 141, 166, 170, 194, 199, 217 and 228. The kit may further comprise reagents necessary for the performance of amplification and detection assay, such as nucleoside triphosphates, nucleic acid polymerase and buffers necessary for the function of the polymerase. In some embodiments, the probe is detectably labeled. In such embodiments, the kit may comprise reagents for labeling and detecting the label.

In yet another embodiment, the invention is a reaction mixture for detecting mutations in the PI3KCA gene, specifically mutation H1047Y and one or more of the mutations selected from H1047L, H1047R, H1047Y, N345K, E542K, E545A, E545G, E545K, G1049R, M1043I, Q546E, Q546L and Q546K as well as a simultaneous query for 1, 2, 3, 4, 5, 6, 7, 8, 9, 10, 11, 12 or 13 of the mutations listed above. The mixture comprises two or more oligonucleotides, wherein one oligonucleotide is SEQ ID NO: 39 and at least one oligonucleotide is selected from SEQ ID NOs: 2, 18, 61, 84, 100, 127, 148, 170, 185, 197, 208, 219 or variations at least 90% identical to and having the 3'-terminal nucleotide of said oligonucleotides, one or more corresponding second primers, and optionally, one or more probes. In variations of this embodiment, the reaction mixture comprises one or more oligonucleotides selected from SEQ ID NOs: 8, 21, 46, 78, 93, 113, 141, 166, 170, 194, 199, 217 and 228. The reaction mixture may further comprise reagents such as nucleoside triphosphates, nucleic acid polymerase and buffers necessary for the function of the polymerase.

In yet another embodiment, the invention is a method of assessing cancer in patient by detecting in a patient's sample mutations in the PI3KCA gene, specifically mutation H1047Y and one or more of the mutations selected from H1047L, H1047R, N345K, E542K, E545A, E545G, E545K, G1049R, M1043I, Q546E, Q546L and Q546K as well as a simultaneous query for 1, 2, 3, 4, 5, 6, 7, 8, 9, 10, 11, 12 or 13 of the mutations listed above, for mutation H1047Y using oligonucleotide SEQ ID NO: 39 and for each of the other mutations using an oligonucleotide selected from SEQ ID NOs: 2, 18, 61, 84, 100, 127, 148, 170, 185, 197, 208, 219 or variations at least 90% identical to and having the 3'-terminal nucleotide of said oligonucleotides. In variations of this embodiment, the oligonucleotides are selected from SEQ ID NOs: 8, 21, 46, 78, 93, 113, 141, 166, 170, 194, 199, 217 and 228.

### Examples

### Exemplary reaction conditions

In all examples below, the following reaction conditions were used. Each reaction included the 10⁴ copies or mutant or wild-type DNA template, 0.1µM each of selective and common primer, detection probe, uracil-N-glycosylase, DNA polymerase and a suitable DNA polymerase buffer. The reactions were subjected to the following thermal cycling profile on the LIGHTCYCLER® 480 instrument (Roche Molecular Diagnostics, Indianapolis, Ind.): 50°C for 5 minutes, followed by 2 cycles of 95°C (10 seconds) to 62°C (30 seconds), and 65 cycles of 93°C (10 seconds) to 62°C (30 seconds). Fluorescence data was collected at the start of each 62°C step. Ct values from each reaction were used to calculate ΔCₜ. Average Ct and standard deviation are shown for each example.

### Example 1

*Performance of primers for detecting mutation H1047L in the human PI3KCA gene*

| SEQ ID NO: | Wt Cₜ | St dev | Mut Cₜ | St dev | Δ Cₜ |
|---|---|---|---|---|---|
| 1 (WT match) | 18.4 | 0.1 | 24.1 | 0.3 | -5.7 |
| 2 | 31.8 | 0.2 | 18.8 | 0.1 | 13.0 |
| 3 | 43.2 | 3.0 | 18.9 | 0.1 | 24.3 |
| 4 | 35.6 | 0.5 | 20.8 | 0.0 | 14.8 |
| 5 | 45.1 | 5.3 | 19.7 | 0.0 | 25.4 |
| 6 | 61.9 | 5.2 | 21.0 | 0.0 | 40.9 |
| 7 | 38.8 | 1.4 | 19.7 | 0.1 | 19.1 |
| 8 | 55.3 | 6.0 | 19.8 | 0.1 | 35.5 |
| 9 | 49.2 | 5.3 | 18.8 | 0.1 | 30.3 |
| 10 | 44.8 | 5.2 | 19.0 | 0.0 | 25.8 |
| 11 | 47.8 | 1.4 | 18.9 | 0.0 | 28.9 |
| 12 | 31.8 | 0.2 | 19.7 | 0.1 | 12.1 |
| 13 | 39.6 | 1.5 | 19.9 | 0.0 | 19.7 |
| 14 | 18.4 | 0.1 | 24.1 | 0.3 | -5.7 |
| 15 | 31.8 | 0.2 | 18.8 | 0.1 | 13.0 |
| 16 | 43.2 | 3.0 | 18.9 | 0.1 | 24.3 |

### Example 2

*Performance of primers for detecting mutation H1047R in the human PI3KCA gene.*

| SEQ ID NO: | Wt Ct | St dev | Mut Ct | St dev | Δ Ct |
|---|---|---|---|---|---|
| 17 (WT match) | 18.2 | 0.2 | 20.4 | 0.0 | -2.2 |
| 18 | 32.2 | 0.3 | 18.9 | 0.0 | 13.3 |
| 19 | 31.7 | 0.3 | 19.5 | 0.0 | 12.2 |
| 20 | 33.9 | 0.4 | 19.5 | 0.0 | 14.4 |
| 21 | 35.5 | 1.0 | 20.1 | 0.3 | 15.4 |
| 22 | 32.7 | 0.7 | 19.4 | 0.1 | 13.3 |
| 23 | 33.7 | 0.6 | 19.5 | 0.0 | 14.2 |
| 24 | 31.9 | 0.4 | 18.9 | 0.0 | 12.9 |
| 25 | 29.8 | 1.0 | 18.9 | 0.1 | 10.9 |
| 26 | 35.4 | 0.7 | 22.3 | 0.1 | 13.2 |
| 27 | 37.1 | 0.6 | 22.8 | 0.0 | 14.3 |
| 28 | 37.0 | 0.7 | 22.7 | 0.0 | 14.3 |
| 29 | 41.7 | 5.1 | 22.3 | 0.1 | 19.4 |
| 30 | 40.2 | 2.4 | 24.7 | 0.2 | 15.5 |
| 31 | 43.4 | 3.2 | 25.1 | 0.5 | 18.3 |
| 32 | 65.0 | 0.0 | 33.5 | 0.0 | 31.5 |
| 33 | 38.8 | 0.9 | 23.1 | 0.3 | 15.8 |
| 34 | 38.4 | 0.7 | 23.1 | 0.3 | 15.2 |
| 35 | 43.0 | 1.8 | 24.6 | 0.5 | 18.5 |
| 36 | 40.4 | 0.0 | 22.7 | 0.2 | 17.8 |
| 37 | 38.3 | 0.9 | 23.2 | 0.0 | 15.1 |

### Example 3

*Performance of primers for detecting mutation H1047Y in the human PI3KCA gene.*

| SEQ ID NO: | Wt Ct | St dev | Mut Ct | St dev | Δ Ct |
|---|---|---|---|---|---|
| 38 (WT match) | 18.0 | 0.2 | 19.3 | 0.0 | -1.3 |
| 39 | 47.5 | 2.9 | 22.6 | 0.0 | 24.9 |
| 40 | 32.8 | 0.3 | 19.3 | 0.0 | 13.5 |
| 41 | 37.3 | 2.4 | 24.6 | 0.1 | 12.7 |
| 42 | 33.2 | 0.4 | 18.9 | 0.0 | 14.3 |
| 43 | 34.4 | 0.8 | 19.4 | 0.0 | 15.0 |
| 44 | 35.0 | 0.9 | 19.1 | 0.2 | 15.9 |
| 45 | 36.1 | 2.4 | 20.2 | 0.0 | 15.9 |
| 46 | 36.0 | 0.4 | 19.5 | 0.2 | 16.5 |
| 47 | 35.8 | 0.8 | 19.5 | 0.1 | 16.3 |
| 48 | 30.0 | 0.2 | 19.6 | 0.1 | 10.4 |
| 49 | 34.4 | 0.5 | 19.7 | 0.0 | 14.7 |
| 50 | 33.3 | 0.3 | 20.4 | 0.0 | 12.9 |
| 51 | 45.8 | 7.9 | 23.3 | 0.0 | 22.5 |
| 52 | 45.1 | 7.6 | 21.8 | 0.1 | 23.4 |
| 53 | 52.9 | 6.5 | 24.1 | 0.1 | 28.8 |
| 54 | 60.8 | 10.4 | 27.2 | 0.0 | 33.5 |
| 55 | 34.7 | 1.2 | 19.3 | 0.0 | 15.4 |
| 56 | 33.1 | 1.3 | 19.8 | 0.0 | 13.3 |
| 57 | 38.2 | 1.3 | 22.9 | 0.7 | 15.3 |
| 58 | 65.0 | 0.0 | 42.5 | 0.3 | 22.5 |
| 59 | 61.6 | 6.1 | 24.6 | 0.1 | 37.0 |

### Example 4

*Performance of primers for detecting mutation N345K in the human PI3KCA gene.*

| SEQ ID NO: | Wt Ct | St dev | Mut Ct | St dev | Δ Ct |
|---|---|---|---|---|---|
| 60 (WT match) | 21.6 | 0.1 | 27.4 | 0.0 | -5.8 |
| 61 | 34.1 | 0.1 | 23.8 | 0.0 | 10.3 |
| 62 | 44.2 | 1.0 | 24.0 | 0.1 | 20.2 |
| 63 | 43.5 | 1.6 | 25.4 | 0.1 | 18.0 |
| 64 | 44.0 | 1.3 | 24.4 | 0.1 | 19.6 |
| 65 | 33.7 | 0.3 | 23.9 | 0.1 | 9.8 |
| 66 | 25.5 | 0.3 | 23.2 | 0.1 | 2.3 |
| 67 | 33.9 | 0.4 | 23.8 | 0.0 | 10.1 |
| 68 | 44.7 | 1.4 | 25.9 | 0.1 | 18.8 |
| 69 | 39.7 | 0.3 | 25.7 | 0.0 | 14.0 |
| 70 | 47.9 | 1.1 | 27.0 | 0.6 | 20.9 |
| 71 | 35.2 | 0.5 | 23.6 | 0.0 | 11.5 |
| 72 | 55.7 | 9.4 | 24.6 | 0.0 | 31.1 |
| 73 | 60.7 | 8.2 | 27.5 | 0.2 | 33.2 |
| 74 | 32.6 | 0.4 | 23.4 | 0.3 | 9.2 |
| 75 | 65.0 | 0.0 | 29.6 | 0.0 | 35.4 |
| 76 | 29.6 | 0.2 | 26.6 | 0.1 | 3.0 |
| 77 | 58.8 | 4.0 | 25.4 | 0.1 | 33.4 |
| 78 | 65.0 | 0.0 | 25.4 | 2.1 | 39.6 |
| 79 | 45.4 | 4.2 | 27.5 | 0.1 | 17.8 |
| 80 | 62.4 | 6.3 | 29.7 | 0.3 | 32.8 |
| 81 | 51.4 | 5.2 | 26.0 | 0.1 | 25.4 |
| 82 | 65.0 | 0.0 | 40.8 | 0.0 | 24.2 |

### Example 5

*Performance of primers for detecting mutation E542K in the human PI3KCA gene*

| SEQ ID NO: | Wt Ct | St dev | Mut Ct | St dev | Δ Ct |
|---|---|---|---|---|---|
| 83 (WT match) | 23.9 | 0.5 | 30.8 | 0.2 | -6.9 |
| 84 | 37.4 | 0.2 | 24.4 | 0.0 | 13.0 |
| 85 | 64.6 | 1.0 | 30.8 | 0.3 | 33.8 |
| 86 | 55.7 | 5.0 | 27.6 | 0.0 | 28.1 |
| 87 | 38.4 | 0.5 | 37.3 | 0.5 | 1.2 |
| 88 | 48.0 | 1.4 | 45.3 | 0.5 | 2.7 |
| 89 | 60.8 | 4.8 | 29.1 | 0.8 | 31.7 |
| 90 | 58.5 | 6.2 | 28.3 | 0.1 | 30.2 |
| 91 | 49.6 | 3.3 | 24.4 | 0.1 | 25.2 |
| 92 | 46.7 | 1.8 | 24.4 | 0.1 | 22.2 |
| 93 | 52.9 | 6.1 | 25.0 | 0.5 | 27.9 |
| 94 | 39.5 | 0.8 | 24.2 | 0.3 | 15.3 |
| 95 | 65.0 | 0.0 | 32.8 | 0.2 | 32.2 |
| 96 | 47.0 | 1.2 | 23.6 | 0.1 | 23.4 |
| 97 | 50.8 | 3.2 | 46.5 | 1.9 | 4.3 |

### Example 6

*Performance of primers for detecting mutation E545A in the human PI3KCA gene*

| SEQ ID NO: | Wt Ct | St dev | Mut Ct | St dev | Δ Ct |
|---|---|---|---|---|---|
| 98 (WT match) | 23.9 | 0.3 | 35.1 | 0.2 | -11.2 |
| 99 | 24.4 | 0.2 | 24.1 | 0.2 | 0.3 |
| 100 | 48.4 | 3.8 | 25.6 | 0.0 | 22.9 |
| 101 | 47.6 | 3.5 | 25.6 | 0.3 | 22.0 |
| 102 | 42.6 | 1.2 | 24.9 | 0.0 | 17.7 |
| 103 | 35.5 | 1.0 | 24.5 | 0.2 | 11.0 |
| 104 | 39.3 | 1.5 | 24.6 | 0.0 | 14.7 |
| 105 | 35.8 | 1.0 | 24.7 | 0.1 | 11.1 |
| 106 | 32.4 | 0.5 | 24.4 | 0.1 | 8.0 |
| 107 | 36.2 | 0.8 | 24.7 | 0.0 | 11.5 |
| 108 | 40.5 | 0.8 | 24.8 | 0.2 | 15.7 |
| 109 | 24.9 | 0.6 | 23.7 | 0.2 | 1.3 |
| 110 | 35.2 | 0.8 | 25.5 | 1.7 | 9.7 |
| 111 | 31.0 | 0.7 | 22.9 | 0.0 | 8.2 |
| 112 | 41.6 | 1.7 | 23.5 | 0.1 | 18.1 |
| 113 | 45.1 | 1.1 | 23.8 | 0.0 | 21.3 |
| 114 | 65.0 | 0.0 | 35.6 | 0.0 | 29.4 |
| 115 | 65.0 | 0.0 | 65.0 | 0.0 | 0.0 |
| 116 | 60.7 | 1.2 | 34.3 | 0.1 | 26.4 |
| 117 | 65.0 | 0.0 | 48.7 | 0.3 | 16.3 |
| 118 | 65.5 | 1.5 | 35.3 | 0.3 | 30.2 |
| 119 | 64.9 | 0.3 | 33.6 | 0.4 | 31.3 |
| 120 | 65.0 | 0.0 | 34.3 | 0.4 | 30.7 |
| 121 | 65.0 | 0.0 | 58.2 | 9.6 | 6.8 |
| 122 | 56.6 | 4.6 | 33.4 | 0.1 | 23.2 |
| 123 | 65.0 | 0.0 | 39.7 | 0.0 | 25.3 |
| 124 | 60.1 | 1.8 | 34.1 | 0.5 | 26.0 |

### Example 7

*Performance of primers for detecting mutation E545G in the human PI3KCA gene*

| SEQ ID NO: | Wt Ct | St dev | Mut Ct | St dev | Δ Ct |
|---|---|---|---|---|---|
| 125 (WT match) | 23.7 | 0.5 | 25.1 | 0.2 | -1.4 |
| 126 | 24.3 | 0.3 | 24.2 | 0.1 | 0.0 |
| 127 | 43.4 | 0.5 | 24.9 | 0.0 | 18.4 |
| 128 | 28.1 | 0.9 | 25.1 | 0.2 | 3.0 |
| 129 | 38.3 | 1.7 | 24.9 | 0.1 | 13.4 |
| 130 | 37.7 | 0.3 | 24.5 | 0.0 | 13.2 |
| 131 | 35.1 | 0.6 | 24.3 | 0.2 | 10.9 |
| 132 | 37.5 | 1.2 | 24.5 | 0.2 | 13.0 |
| 133 | 29.8 | 0.5 | 24.3 | 0.1 | 5.5 |
| 134 | 35.9 | 0.3 | 24.6 | 0.1 | 11.3 |
| 135 | 33.6 | 0.6 | 24.2 | 0.3 | 9.5 |
| 136 | 24.5 | 0.4 | 23.7 | 0.1 | 0.8 |
| 137 | 31.0 | 0.4 | 25.8 | 1.5 | 5.2 |
| 138 | 54.3 | 7.8 | 25.4 | 0.3 | 28.9 |
| 139 | 65.0 | 0.0 | 39.0 | 0.0 | 26.0 |
| 140 | 56.3 | 4.7 | 30.0 | 0.2 | 26.3 |
| 141 | 43.9 | 0.9 | 23.7 | 0.3 | 20.3 |
| 142 | 43.8 | 1.1 | 24.1 | 0.3 | 19.7 |
| 143 | 65.0 | 0.0 | 39.2 | 0.4 | 25.8 |
| 144 | 30.2 | 1.0 | 26.3 | 0.0 | 4.0 |
| 145 | 65.0 | 0.0 | 65.0 | 0.0 | 0.0 |
| 146 | 37.9 | 0.7 | 23.8 | 0.2 | 14.1 |

### Example 8

*Performance of primers for detecting mutation E545K in the human PI3KCA gene*

| SEQ ID NO: | Wt Ct | St dev | Mut Ct | St dev | Δ Ct |
|---|---|---|---|---|---|
| 147 (WT match) | 23.5 | 0.3 | 25.4 | 0.2 | -1.9 |
| 148 | 29.1 | 0.5 | 23.9 | 0.0 | 5.2 |
| 149 | 45.7 | 2.7 | 26.4 | 0.0 | 19.3 |
| 150 | 42.1 | 1.1 | 24.9 | 0.1 | 17.2 |
| 151 | 26.5 | 0.5 | 30.9 | 0.1 | -4.3 |
| 152 | 40.7 | 1.7 | 24.4 | 0.1 | 16.3 |
| 153 | 47.3 | 2.3 | 26.7 | 0.0 | 20.6 |
| 154 | 44.7 | 2.0 | 25.7 | 0.2 | 19.0 |
| 155 | 37.9 | 0.9 | 24.0 | 0.3 | 13.9 |
| 156 | 35.8 | 0.5 | 24.1 | 0.2 | 11.7 |
| 157 | 41.6 | 1.1 | 24.1 | 0.2 | 17.5 |
| 158 | 31.2 | 0.7 | 24.9 | 0.0 | 6.3 |
| 159 | 56.3 | 1.6 | 34.5 | 0.4 | 21.9 |
| 160 | 28.2 | 0.6 | 22.9 | 0.4 | 5.3 |
| 161 | 45.4 | 1.5 | 25.6 | 0.3 | 19.8 |
| 162 | 51.2 | 5.7 | 29.8 | 0.2 | 21.4 |
| 163 | 58.7 | 7.0 | 32.0 | 0.0 | 26.8 |
| 164 | 48.7 | 2.7 | 29.5 | 0.1 | 19.2 |
| 165 | 49.5 | 7.1 | 30.5 | 0.4 | 19.0 |
| 166 | 42.0 | 2.0 | 24.5 | 0.0 | 17.5 |

### Example 9

*Performance of primers for detecting mutation Q546K in the human PI3KCA gene*

| SEQ ID NO: | Wt Ct | St dev | Mut Ct | St dev | Δ Ct |
|---|---|---|---|---|---|
| 167 (WT match) | 23.3 | 0.5 | 25.8 | 0.1 | -2.5 |
| 168 | 34.9 | 0.4 | 41.8 | 0.6 | -6.9 |
| 169 | 26.2 | 0.6 | 28.4 | 0.0 | -2.2 |
| 170 | 55.2 | 2.8 | 25.9 | 0.0 | 29.2 |
| 171 | 44.6 | 1.8 | 26.7 | 0.1 | 17.9 |
| 172 | 42.1 | 1.0 | 24.6 | 0.3 | 17.4 |
| 173 | 37.2 | 1.1 | 25.3 | 0.1 | 11.9 |
| 174 | 35.3 | 1.3 | 24.5 | 0.0 | 10.7 |
| 175 | 65.0 | 0.0 | 65.0 | 0.0 | 0.0 |
| 176 | 44.7 | 0.9 | 26.6 | 0.2 | 18.1 |
| 177 | 41.5 | 0.4 | 26.0 | 0.2 | 15.6 |
| 178 | 37.4 | 0.9 | 44.5 | 0.3 | -7.0 |
| 179 | 65.0 | 0.0 | 65.0 | 0.0 | 0.0 |
| 180 | 65.0 | 0.0 | 65.0 | 0.0 | 0.0 |
| 181 | 48.9 | 2.9 | 25.2 | 0.1 | 23.7 |
| 182 | 33.4 | 0.3 | 24.4 | 0.0 | 9.0 |
| 183 | 40.1 | 1.1 | 25.8 | 0.1 | 14.3 |

### Example 10

*Performance of primers for detecting mutation Q546E in the human PI3KCA gene*

| SEQ ID NO: | Wt Ct | St dev | Mut Ct | St dev | Δ Ct |
|---|---|---|---|---|---|
| 184 (WT match) | 21.5 | 0.4 | 31.9 | 0.5 | -10.4 |
| 185 | 30.8 | 0.5 | 22.6 | 0.0 | 8.2 |
| 186 | 36.7 | 1.2 | 24.6 | 0.1 | 12.1 |
| 187 | 39.0 | 0.6 | 27.8 | 0.0 | 11.1 |
| 188 | 56.8 | 5.8 | 27.6 | 0.4 | 29.2 |
| 189 | 38.3 | 0.7 | 23.5 | 0.1 | 14.8 |
| 190 | 35.0 | 0.4 | 23.9 | 0.1 | 11.2 |
| 191 | 43.7 | 0.8 | 23.8 | 0.1 | 19.9 |
| 192 | 50.5 | 3.8 | 25.3 | 0.4 | 25.3 |
| 193 | 38.3 | 0.8 | 26.4 | 0.1 | 11.8 |
| 194 | 52.7 | 3.1 | 24.7 | 0.1 | 28.1 |

### Example 11

*Performance of primers for detecting mutation Q546L in the human PI3KCA gene*

| SEQ ID NO: | Wt Ct | St dev | Mut Ct | St dev | Δ Ct |
|---|---|---|---|---|---|
| 195 (WT match) | 22.2 | 0.4 | 33.9 | 0.1 | -11.7 |
| 197 | 31.2 | 0.7 | 21.9 | 0.1 | 9.3 |
| 198 | 63.0 | 2.5 | 29.5 | 0.1 | 33.5 |
| 199 | 56.5 | 3.0 | 23.2 | 0.3 | 33.3 |
| 200 | 47.1 | 2.3 | 22.8 | 0.0 | 24.2 |
| 201 | 56.4 | 5.6 | 23.1 | 0.2 | 33.3 |
| 202 | 51.9 | 4.6 | 24.3 | 0.2 | 27.6 |
| 203 | 55.9 | 2.6 | 23.5 | 0.2 | 32.4 |
| 204 | 42.4 | 0.6 | 22.6 | 0.1 | 19.8 |
| 205 | 39.7 | 0.9 | 22.4 | 0.4 | 17.3 |
| 206 | 44.8 | 1.7 | 22.4 | 0.2 | 22.4 |

### Example 12

*Performance of primers for detecting mutation G1049R in the human PI3KCA gene*

| SEQ ID NO: | Wt Ct | St dev | Mut Ct | St dev | Δ Ct |
|---|---|---|---|---|---|
| 207 (WT match) | 18.8 | 0.1 | 30.3 | 0.1 | -11.5 |
| 208 | 32.2 | 0.4 | 20.4 | 0.0 | 11.8 |
| 209 | 36.1 | 0.5 | 22.3 | 0.1 | 13.8 |
| 210 | 41.6 | 2.1 | 20.7 | 0.1 | 20.9 |
| 211 | 43.4 | 1.6 | 26.4 | 0.0 | 16.9 |
| 212 | 49.9 | 8.2 | 20.1 | 0.3 | 29.8 |
| 213 | 48.3 | 5.6 | 20.4 | 0.1 | 28.0 |
| 214 | 41.3 | 2.2 | 20.8 | 0.0 | 20.5 |
| 215 | 43.5 | 4.9 | 20.8 | 0.1 | 22.8 |
| 216 | 48.3 | 2.9 | 20.7 | 0.1 | 27.7 |
| 217 | 53.0 | 5.1 | 20.5 | 0.1 | 32.5 |

### Example 13

*Performance of primers for detecting mutation M1043I in the human PI3KCA gene*

| SEQ ID NO: | Wt Ct | St dev | Mut Ct | St dev | Δ Ct |
|---|---|---|---|---|---|
| 218 | 17.8 | 0.1 | 30.8 | 0.0 | -13.0 |
| 219 | 46.7 | 4.2 | 19.5 | 0.1 | 27.1 |
| 220 | 53.8 | 6.5 | 26.7 | 0.0 | 27.1 |
| 221 | 51.0 | 9.1 | 32.1 | 0.3 | 19.0 |
| 222 | 38.7 | 2.5 | 22.0 | 0.0 | 16.7 |
| 223 | 52.1 | 5.0 | 21.7 | 0.0 | 30.4 |
| 224 | 50.6 | 5.2 | 25.6 | 0.0 | 25.0 |
| 225 | 51.5 | 9.1 | 20.5 | 0.0 | 31.0 |
| 226 | 52.0 | 2.5 | 19.7 | 0.0 | 32.3 |
| 227 | 50.0 | 5.0 | 20.5 | 0.0 | 29.5 |
| 228 | 61.5 | 5.8 | 20.1 | 0.0 | 41.3 |
| 229 | 65.0 | 0.0 | 35.5 | 0.0 | 29.5 |
| 230 | 65.0 | 0.0 | 23.2 | 0.1 | 41.8 |

While the invention has been described in detail with reference to specific examples, it will be apparent to one skilled in the art that various modifications can be made within the scope of this invention. Thus the scope of the invention should not be limited by the examples described herein, but by the claims presented below.

## Claims

1. An oligonucleotide for detecting a mutation H1047Y in the PIK3CA gene being at least 90% identical to and having the 3'-terminal nucleotide of SEQ ID NO: 39, which comprises 3 or fewer mismatches, excluding the 3'-terminal nucleotide, wherein at least one mismatch or at least one modified nucleotide is among the penultimate 5 nucleotides at the 3'-terminus.

2. A method of assaying a sample for the presence of mutation H1047Y in the human PIK3CA gene comprising contacting the sample with one allele-specific nucleotide oligonucleotide sharing at least 90% identity with and having the same 3-terminal nucleotide as an oligonucleotide comprising SEQ ID NO: 39, which comprises 3 or fewer mismatches, excluding the 3'-terminal nucleotide, wherein at least one mismatch or at least one modified nucleotide is among the penultimate 5 nucleotides at the 3'-terminus.

3. The method of claim 2, wherein the oligonucleotide is SEQ ID NO: 46.

4. The method of claim 2 or 3, further comprising at least one allele-specific oligonucleotide for each mutation other than H1047Y sharing at least 90% identity with and having the same 3'-terminal nucleotide as an oligonucleotide selected from a group consisting of SEQ ID NOs: 2, 18, 61, 84, 100, 127, 148, 170, 185, 197, 208 and 219.

5. The method of claim 4, wherein the oligonucleotide is selected from SEQ ID NOs: 8, 21, 78, 93, 113, 141, 166, 170, 194, 199, 217 and 228.

6. A set of oligonucleotides for detecting mutation H1047Y and one or more of mutations H1047L, H1047R, N345K, E542K, E545A, E545G, E545K, G1049R, M1043I, Q546E, Q546L and Q546K in the PIK3CA gene comprising a combination of two or more oligonucleotides, wherein one oligonucleotide is identical with an oligonucleotide of claim 1 and at least one allele-specific oligonucleotide for each mutation other than H1047Y is sharing at least 90% identity with and having the same 3-terminal nucleotide as an oligonucleotide selected from a group consisting of SEQ ID NOs: 2, 18, 61, 84, 100, 127, 148, 170, 185, 197, 208 and 219.

7. The set of claim 6, wherein the oligonucleotide is SEQ ID NO: 46 and/or the allele-specific oligonucleotide is selected from a group consisting of SEQ ID NOs: 8, 21, 78, 93, 113, 141, 166, 170, 194, 199, 217 and 228.

8. The set of claim 6 comprising SEQ ID NOs: 8, 21, 46, 78, 93, 113, 141, 166, 170, 194, 199, 217 and 228.

9. A reaction mixture for detecting mutation H1047Y and one or more mutations H1047L, H1047R, N345K, E542K, E545A, E545G, E454K, G1049R, M1043I, Q546E, Q546L and Q546K in the PIK3CA gene comprising one oligonucleotide identical with an oligonucleotide of claim 1 and at least one allele-specific oligonucleotide for each mutation other than H1047Y sharing at least 90% identity with and having the same 3'-terminal nucleotide as an oligonucleotide selected from a group consisting of SEQ ID NOs: 2, 18, 61, 84, 100, 127, 148, 170, 185, 197, 208, 219.

10. The reaction mixture of claim 9, wherein the oligonucleotide is SEQ ID NO: 46 and/or the allele-specific oligonucleotide is selected from a group consisting of SEQ ID NOs: 8, 21, 78, 93, 113, 141, 166, 170, 194, 199, 217 and 228.

11. The reaction mixture of claim 9, comprising SEQ ID NOs: 8, 21, 46, 78, 93, 113, 141, 166, 170, 194, 199, 217 and 228.

12. A method of assessing cancer in a patient by detecting in the patient's sample mutation H1047Y and one or more of the mutations H1047L, H1047R, N345K, E542K, E545A, E545G, E545K, G1049R, M1043I, Q546E, Q546L and Q546K in the PIK3CA gene comprising contacting the sample with one oligonucleotide identical with an oligonucleotide of claim 1 and one allele-specific nucleotide oligonucleotide for each mutation other than H1047Y sharing at least 90% identity with and having the same 3'-terminal nucleotide as an oligonucleotide selected from a group consisting of SEQ ID NOs: 2, 18, 61, 84, 100, 127, 148, 170, 185, 197, 208, 219.

13. The method of claim 12, wherein the oligonucleotide is SEQ ID NO: 46 and/or the allele-specific oligonucleotide is selected from a group consisting of SEQ ID NOs: 8, 21, 78, 93, 113, 141, 166, 170, 194, 199, 217 and 228.

## Patentansprüche

1. Oligonukleotid zum Nachweis einer H1047Y-Mutation im PIK3CA-Gen, das zu mindestens 90 % mit SEQ ID NO: 39 identisch ist und deren 3'-terminales Nukleotid aufweist und das 3 oder weniger Fehlpaarungen, das 3'-terminale Nukleotid ausgeschlossen, umfasst, wobei mindestens eine Fehlpaarung oder mindestens ein modifiziertes Nukleotid unter den vorletzten 5 Nukleotiden am 3'-Terminus ist.

2. Verfahren zum Untersuchen einer Probe auf das Vorhandensein einer H1047Y-Mutation im menschlichen PIK3CA-Gen, umfassend das Inkontaktbringen der Probe mit einem allelspezifischen Nukleotid-Oligonukleotid, das zu mindestens 90 % mit einem Oligonukleotid umfassend SEQ ID NO: 39 identisch ist und das gleiche 3'-terminale Nukleotid wie dieses aufweist und das 3 oder weniger Fehlpaarungen, das 3'-terminale Nukleotid ausgeschlossen, umfasst, wobei mindestens eine Fehlpaarung oder mindestens ein modifiziertes Nukleotid unter den vorletzten 5 Nukleotiden am 3'-Terminus ist.

3. Verfahren nach Anspruch 2, wobei es sich bei dem Oligonukleotid um SEQ ID NO: 46 handelt.

4. Verfahren nach Anspruch 2 oder 3, ferner umfassend mindestens ein allelspezifisches Oligonukleotid für jede Mutation, bei der es sich nicht um H1047Y handelt, das zu mindestens 90 % mit einem Oligonukleotid ausgewählt aus einer Gruppe bestehend aus SEQ ID NO: 2, 18, 61, 84, 100, 127, 148, 170, 185, 197, 208 und 219 identisch ist und das gleiche 3'-terminale Nukleotid wie dieses aufweist.

5. Verfahren nach Anspruch 4, wobei das Oligonukleotid aus SEQ ID NO: 8, 21, 78, 93, 113, 141, 166, 170, 194, 199, 217 und 228 ausgewählt ist.

6. Satz von Oligonukleotiden zum Nachweis einer H1047Y-Mutation und von einer oder mehreren der Mutationen H1047L, H1047R, N345K, E542K, E545A, E545G, E545K, G1049R, M1043I, Q546E, Q546L und Q546K im PIK3CA-Gen, umfassend eine Kombination von zwei oder mehr Oligonukleotiden, wobei ein Oligonukleotid identisch mit einem Oligonukleotid nach Anspruch 1 ist und mindestens ein allelspezifisches Oligonukleotid für jede Mutation, bei der es sich nicht um H1047Y handelt, zu mindestens 90 % mit einem Oligonukleotid ausgewählt aus einer Gruppe bestehend aus SEQ ID NO: 2, 18, 61, 84, 100, 127, 148, 170, 185, 197, 208 und 219 identisch ist und das gleiche 3'-terminale Nukleotid wie dieses aufweist.

7. Satz nach Anspruch 6, wobei es sich bei dem Oligonukleotid um SEQ ID NO: 46 handelt und/oder das allelspezifische Oligonukleotid aus einer Gruppe bestehend aus SEQ ID NO: 8, 21, 78, 93, 113, 141, 166, 170, 194, 199, 217 und 228 ausgewählt ist.

8. Satz nach Anspruch 6, umfassend SEQ ID NO: 8, 21, 46, 78, 93, 113, 141, 166, 170, 194, 199, 217 und 228.

9. Reaktionsgemisch zum Nachweis einer H1047Y-Mutation und von einer oder mehreren der Mutationen H1047L, H1047R, N345K, E542K, E545A, E545G, E454K, G1049R, M1043I, Q546E, Q546L und Q546K im PIK3CA-Gen, umfassend ein mit einem Oligonukleotid nach Anspruch 1 identisches Oligonukleotid und mindestens ein allelspezifisches Oligonukleotid für jede Mutation, bei der es sich nicht um H1047Y handelt, das zu mindestens 90 % mit einem Oligonukleotid ausgewählt aus einer Gruppe bestehend aus SEQ ID NO: 2, 18, 61, 84, 100, 127, 148, 170, 185, 197, 208, 219 identisch ist und das gleiche 3'-terminale Nukleotid wie dieses aufweist.

10. Reaktionsgemisch nach Anspruch 9, wobei es sich bei dem Oligonukleotid um SEQ ID NO: 46 handelt und/oder das allelspezifische Oligonukleotid aus der Gruppe bestehend aus SEQ ID NO: 8, 21, 78, 93, 113, 141, 166, 170, 194, 199, 217 und 228 ausgewählt ist.

11. Reaktionsgemisch nach Anspruch 9, umfassend SEQ ID NO: 8, 21, 46, 78, 93, 113, 141, 166, 170, 194, 199, 217 und 228.

12. Verfahren zur Feststellung von Krebs in einem Patienten durch Nachweis einer H1047Y-Mutation und von einer oder mehreren der Mutationen H1047L, H1047R, N345K, E542K, E545A, E545G, E545K, G1049R, M10431, Q546E, Q546L und Q546K im PIK3CA-Gen in der Probe des Patienten, umfassend das Inkontaktbringen der Probe mit einem mit einem Oligonukleotid nach Anspruch 1 identischen Oligonukleotid und einem allelspezifischen Nukleotid-Oligonukleotid für jede Mutation, bei der es sich nicht um H1047Y handelt, das zu mindestens 90 % mit einem Oligonukleotid ausgewählt aus einer Gruppe bestehend aus SEQ ID NO: 2, 18, 61, 84, 100, 127, 148, 170, 185, 197, 208, 219 identisch ist und das gleiche 3'-terminale Nukleotid wie dieses aufweist.

13. Verfahren nach Anspruch 12, wobei es sich bei dem Oligonukleotid um SEQ ID NO: 46 handelt und/oder das allelspezifische Oligonukleotid aus der Gruppe bestehend aus SEQ ID NO: 8, 21, 78, 93, 113, 141, 166, 170, 194, 199, 217 und 228 ausgewählt ist.

## Revendications

1. Oligonucléotide pour la détection d'une mutation H1047Y dans le gène PIK3CA qui est au moins 90 % identique à et comporte le nucléotide 3'-terminal de SEQ ID NO: 39, qui comprend 3 mésappariements ou moins, à l'exclusion du nucléotide 3'-terminal, dans lequel au moins un mésappariement ou au moins un nucléotide modifié fait partie des 5 pénultièmes nucléotides à l'extrémité 3'.

2. Procédé d'analyse d'un échantillon pour la présence d'une mutation H1047Y dans le gène PIK3CA humain comprenant la mise en contact de l'échantillon avec un oligonucléotide nucléotidique spécifique d'un allèle partageant une identité d'au moins 90 % avec et comportant le même nucléotide 3-terminal qu'un oligonucléotide comprenant la SEQ ID NO: 39, qui comprend 3 mésappariements ou moins, à l'exclusion du nucléotide 3'-terminal, dans lequel au moins un mésappariement ou au moins un nucléotide modifié fait partie des 5 pénultièmes nucléotides à l'extrémité 3'.

3. Procédé selon la revendication 2, dans lequel l'oligonucléotide est la SEQ ID NO: 46.

4. Procédé selon la revendication 2 ou 3, comprenant en outre au moins un oligonucléotide spécifique d'un allèle pour chaque mutation autre que H1047Y partageant une identité d'au moins 90 % avec et comportant le même nucléotide 3'-terminal qu'un oligonucléotide choisi dans le groupe consistant en les SEQ ID NO: 2, 18, 61, 84, 100, 127, 148, 170, 185, 197, 208 et 219.

5. Procédé selon la revendication 4, dans lequel l'oligonucléotide est choisi parmi les SEQ ID NO: 8, 21, 78, 93, 113, 141, 166, 170, 194, 199, 217 et 228.

6. Jeu d'oligonucléotides pour la détection de la mutation H1047Y et d'une ou plusieurs mutations H1047L, H1047R, N345K, E542K, E545A, E545G, E545K, G1049R, M1043I, Q546E, Q546L et Q546K dans le gène PIK3CA comprenant une association de deux oligonucléotides ou plus, dans lequel un oligonucléotide est identique à un oligonucléotide de la revendication 1 et au moins un oligonucléotide spécifique d'un allèle pour chaque mutation autre que H1047Y partage une identité d'au moins 90 % avec et comporte le même nucléotide 3'-terminal qu'un oligonucléotide choisi dans le groupe consistant en les SEQ ID NO: 2, 18, 61, 84, 100, 127, 148, 170, 185, 197, 208 et 219.

7. Jeu selon la revendication 6, dans lequel l'oligonucléotide est la SEQ ID NO: 46 et/ou l'oligonucléotide spécifique d'un allèle est choisi dans le groupe consistant en les SEQ ID NO: 8, 21, 78, 93, 113, 141, 166, 170, 194, 199, 217 et 228.

8. Jeu selon la revendication 6, comprenant les SEQ ID NO: 8, 21, 46, 78, 93, 113, 141, 166, 170, 194, 199, 217 et 228.

9. Mélange réactionnel pour la détection de la mutation H1047Y et d'une ou plusieurs mutations H1047L, H1047R, N345K, E542K, E545A, E545G, E545K, G1049R, M1043I, Q546E, Q546L et Q546K dans le gène PIK3CA comprenant un oligonucléotide identique à un oligonucléotide de la revendication 1 et au moins un oligonucléotide spécifique d'un allèle pour chaque mutation autre que H1047Y partageant une identité d'au moins 90 % avec et comportant le même nucléotide 3'-terminal qu'un oligonucléotide choisi dans le groupe consistant en les SEQ ID NO: 2, 18, 61, 84, 100, 127, 148, 170, 185, 197, 208, 219.

10. Mélange réactionnel selon la revendication 9, dans lequel l'oligonucléotide est la SEQ ID NO: 46 et/ou l'oligonucléotide spécifique d'un allèle est choisi dans le groupe consistant en les SEQ ID NO: 8, 21, 78, 93, 113, 141, 166, 170, 194, 199, 217 et 228.

11. Mélange réactionnel selon la revendication 9, comprenant les SEQ ID NO: 8, 21, 46, 78, 93, 113, 141, 166, 170, 194, 199, 217 et 228.

12. Procédé d'évaluation d'un cancer chez un patient par la détection dans l'échantillon du patient de la mutation H1047Y et d'une ou plusieurs des mutations H1047L, H1047R, N345K, E542K, E545A, E545G, E545K, G1049R, M1043I, Q546E, Q546L et Q546K dans le gène PIK3CA comprenant la mise en contact de l'échantillon avec un oligonucléotide identique à un oligonucléotide de la revendication 1 et au moins un oligonucléotide spécifique d'un allèle pour chaque mutation autre que H1047Y partageant une identité d'au moins 90 % avec et comportant le même nucléotide 3'-terminal qu'un oligonucléotide choisi dans le groupe consistant en les SEQ ID NO: 2, 18, 61, 84, 100, 127, 148, 170, 185, 197, 208, 219.

13. Procédé selon la revendication 12, dans lequel l'oligonucléotide est la SEQ ID NO: 46 et/ou l'oligonucléotide spécifique d'un allèle est choisi dans le groupe consistant en les SEQ ID NO: 8, 21, 78, 93, 113, 141, 166, 170, 194, 199, 217 et 228.
